(19) **Europäisches Patentamt**
**European Patent Office**
**Office européen des brevets**

(11) **EP 3 535 678 B1**

(12) **EUROPEAN PATENT SPECIFICATION**

(45) Date of publication and mention of the grant of the patent:
**22.12.2021 Bulletin 2021/51**

(21) Application number: **17804368.3**

(22) Date of filing: **02.11.2017**

(51) Int Cl.:
***G16B 25/00*** (2019.01)

(86) International application number:
**PCT/US2017/059785**

(87) International publication number:
**WO 2018/085585 (11.05.2018 Gazette 2018/19)**

(54) **SYSTEMS AND METHODS FOR OUTLIER SIGNIFICANCE ASSESSMENT**

SYSTEME UND VERFAHREN ZU BEWERTUNG DER SIGNIFIKANZ EINES AUSREISSERS

SYSTÈMES ET PROCÉDÉS D'ÉVALUATION DE LA SIGNIFICATION DE VALEURS ABERRANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2016 US 201662417149 P**

(43) Date of publication of application:
**11.09.2019 Bulletin 2019/37**

(73) Proprietor: **Illumina, Inc.**
**San Diego, CA 92122 (US)**

(72) Inventors:
• **NG, Sam**
**Santa Clara, California 95050 (US)**
• **GAO, Hong**
**Santa Clara, California 95050 (US)**
• **GIERMAN, Hendrikus Jasper**
**Santa Clara, California 95050 (US)**

(74) Representative: **Mewburn Ellis LLP**
**Aurora Building**
**Counterslip**
**Bristol BS1 6BX (GB)**

(56) References cited:
• **GAO H ET AL: "Abstract 4552: ssCOPA: a novel method with improved sensitivity to assess statistical significance for cancer outlier profile analysis of gene expression", CANCER RESEARCH SUPPLEMENT, AACR Annual Meeting 2017; April 1-5, 2017, Washington, DC, 1 April 2017 (2017-04-01), pages 1-2, XP055458575, Washington, DC Retrieved from the Internet: URL:http://cancerres.aacrjournals.org/cont ent/77/13_Supplement/4552.short [retrieved on 2018-03-12]**
• **RHODES D R ET AL: "AGTR1 overexpression defines a subset of breast cancer and confers sensitivity to losartan, an AGTR1 antagonist", PROCEEDINGS OF THE NATIONAL ACADEMY OF SCIENCES (PNAS) USA, vol. 106, no. 25, 23 June 2009 (2009-06-23), pages 10284-10289, XP002592235, ISSN: 0027-8424, DOI: 10.1073/PNAS.0900351106 [retrieved on 2009-06-01] cited in the application -& RHODES D R ET AL: "Supporting information. AGTR1 overexpression defines a subset of breast cancer and confers sensitivity to losartan, an AGTR1 antagonist", Proceedings of the National Academy of Sciences, 23 June 2009 (2009-06-23), pages 10284-10289, XP055459317, DOI: 10.1073/pnas.0900351106 Retrieved from the Internet: URL:http://www.pnas.org/content/pnas/suppl /2009/06/01/0900351106.DCSupplemental/0900 351106SI.pdf [retrieved on 2018-03-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- TOMLINS S A ET AL: "The Role of SPINK1 in ETS Rearrangement-Negative Prostate Cancers", CANCER CELL, vol. 13, no. 6, 1 June 2008 (2008-06-01), pages 519-528, XP055458894, US ISSN: 1535-6108, DOI: 10.1016/j.ccr.2008.04.016 -& TOMLINS S A ET AL: "Supplemental Data. The Role of SPINK1 in ETS Rearrangement-Negative Prostate Cancers", CANCER CELL, 1 January 2008 (2008-01-01), pages 519-528, XP055459148, United States DOI: 10.1016/j.ccr.2008.04.016 Retrieved from the Internet: URL:http://www.cell.com/cms/attachment/598 837/4705943/mmc1.pdf [retrieved on 2018-03-14]
- PARK I ET AL: "Mining cancer genes with running-sum statistics", DATA AND TEXT MINING IN BIOINFORMATICS, ACM, 2 PENN PLAZA, SUITE 701 NEW YORK NY 10121-0701 USA, 6 November 2009 (2009-11-06), pages 35-42, XP058242826, DOI: 10.1145/1651318.1651326 ISBN: 978-1-60558-803-2
- TIBSHIRANI R ET AL: "Outlier sums for differential gene expression analysis", BIOSTATISTICS, vol. 8, no. 1, 15 May 2006 (2006-05-15), pages 2-8, XP055458814, GB ISSN: 1465-4644, DOI: 10.1093/biostatistics/kxl005
- OCHS M F ET AL: "Outlier analysis and top scoring pair for integrated data analysis and biomarker discovery", IEEE/ACM TRANSACTIONS ON COMPUTATIONAL BIOLOGY AND BIOINFORMATICS, IEEE SERVICE CENTER, NEW YORK, NY, US, vol. 11, no. 3, 1 May 2014 (2014-05-01), pages 520-532, XP058058117, ISSN: 1545-5963, DOI: 10.1109/TCBB.2013.153
- PAWLIKOWSKA I ET AL: "The most informative spacing test effectively discovers biologically relevant outliers or multiple modes in expression", BIOINFORMATICS., vol. 30, no. 10, 15 May 2014 (2014-05-15), pages 1400-1408, XP055459039, GB ISSN: 1367-4803, DOI: 10.1093/bioinformatics/btu039
- MPINDI J P ET AL: "GTI: A Novel Algorithm for Identifying Outlier Gene Expression Profiles from Integrated Microarray Datasets", PLOS ONE, vol. 6, no. 2, 18 February 2011 (2011-02-18), page e17259, XP055458649, DOI: 10.1371/journal.pone.0017259
- WANG Y ET AL: "LSOSS: Detection of Cancer Outlier Differential Gene Expression", BIOMARKER INSIGHTS, vol. 5, 1 January 2010 (2010-01-01), XP055458623, New Zealand ISSN: 1177-2719, DOI: 10.4137/BMI.S5175
- HARVEY R C ET AL: "Identification of novel cluster groups in pediatric high-risk B-precursor acute lymphoblastic leukemia with gene expression profiling: correlation with genome-wide DNA copy number alterations, clinical characteristics, and outcome", BLOOD, vol. 116, no. 23, 2 December 2010 (2010-12-02), pages 4874-4884, XP055073198, ISSN: 0006-4971, DOI: 10.1182/blood-2009-08-239681 -& HARVEY R C ET AL: "Supplemental materials. Identification of novel cluster groups in pediatric high-risk B-precursor acute lymphoblastic leukemia with gene expression profiling: correlation with genome-wide DNA copy number alterations, clinical characteristics, and outcome", Blood, 2 December 2010 (2010-12-02), pages 4874-4884, XP055459268, Retrieved from the Internet: URL:http://www.bloodjournal.org/content/bl oodjournal/suppl/2010/08/10/blood-2009-08-239681.DC1/Document1.pdf?sso-checked=true [retrieve
- WU B: "Cancer outlier differential gene expression detection", BIOSTATISTICS, vol. 8, no. 3, 4 October 2006 (2006-10-04), pages 566-575, XP055458848, GB ISSN: 1465-4644, DOI: 10.1093/biostatistics/kxl029
- WANG Y ET AL: "Identifying novel prostate cancer associated pathways based on integrative microarray data analysis", PMC /COMPUTATIONAL BIOLOGY AND CHEMISTRY/, ELSEVIER, AMSTERDAM, NL, vol. 35, no. 3, 16 April 2011 (2011-04-16), pages 151-158, XP028231094, ISSN: 1476-9271, DOI: 10.1016/J.COMPBIOLCHEM.2011.04.003 [retrieved on 2011-04-27]
- VAN DER LAAN M J ET AL: "Gene expression analysis with the parametric bootstrap", BIOSTATISTICS, OXOFRD UNIVERSITY PRESS, OXFORD, GB, vol. 2, no. 4, 1 December 2001 (2001-12-01), pages 445-461, XP001207769, ISSN: 1465-4644, DOI: 10.1093/BIOSTATISTICS/2.4.445

**Description**

**TECHNICAL FIELD**

The disclosed systems and methods concern identification of outliers. More specifically, the disclosed systems and methods concern improved methods for determining the significance of factors in continuous-valued observations in analyses comprising samples, the samples including observational data corresponding to factors.

**BACKGROUND**

Outlier analysis may enable determination of outliers in gene expression observational data. However, current outlier analysis is not directly comparable across different analyses or under different input parameters. This limits integrating results across analyses in meta-analysis. Also, current methods of meta-analysis may require a large number of analyses, and may not enable adjustment of results based on the different sizes of the analyses. There is therefore room for improvement.

**SUMMARY**

A detection system for identifying genes with outlier expression across multiple samples, includes :

at least one processor; and at least one non-transitory computer readable medium containing instructions. The instructions, when executed by the at least one processor, cause the at least one processor to perform operations comprising: receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene;

determining a null distribution of the distribution statistic using the standardized gene expression data; and outputting a significance value of the genes across the multiple samples, the significance value based on the value of the distribution statistic and the null distribution.

A non-transitory computer readable medium for identifying genes with outlier expression across multiple samples includes instructions that, when executed by the at least one processor, cause the at least one processor to perform operations. These operations include: receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene; determining a null distribution of the distribution statistic using the standardized gene expression data; and outputting a significance value of the genes across the multiple samples, the significance value based on the value of the distribution statistic and the null distribution.

A computer-implemented method for identifying genes with outlier expression across multiple samples, includes : receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene; determining a null distribution of the distribution statistic using the standardized gene expression data; and outputting a significance value of the genes across the multiple samples, the significance value based on the value of the distribution statistic and the null distribution.

It is to be understood that both the foregoing general description and the following detailed description are exemplary and explanatory only and are not restrictive of the disclosed embodiments, as claimed.

## BRIEF DESCRIPTION OF THE DRAWINGS

The drawings are not necessarily to scale or exhaustive. Instead, emphasis is generally placed upon illustrating the principles of the inventions described herein. The accompanying drawings, which are incorporated in and constitute a part of this specification, illustrate several embodiments consistent with the disclosure and, together with the description, serve to explain the principles of the disclosure. In the drawings:

Fig. 1 depicts an exemplary schematic of a system for determining the significance of outliers in observational data.

Figs. 2A, 2B, and 2C depict exemplary flowcharts of methods for determining the significance of outliers in observational data.

Figs. 3A and 3B depict an exemplary illustration of null distributions estimated according to the envisioned systems and methods.

Fig. 3C depicts a comparison between two exemplary methods for determining the significance of outliers in observational data using a null distribution.

Fig. 4A depicts an exemplary flowchart of a method for determining the significance of outliers in observational data using Bernoulli trials.

Fig. 4B is an exemplary conceptual diagram illustrating features of determining the significance of outliers in observational data using Bernoulli trials.

Fig. 5 depicts a comparison between two exemplary methods for determining the significance of outliers in observational data.

Fig. 6 depicts an exemplary flowchart of a method for performing a modified rank-product test meta-analysis.

Figs. 7A and 7B depict exemplary computing systems suitable for implementing disclosed systems and methods.

Fig. 8 depicts a bootstrap resampling technique, according to embodiments of the invention.

Fig. 9 depicts a bootstrap resampling technique fitted to a function, according to embodiments of the invention.

Fig. 10 depicts a binomial probability technique for measuring outlier observation data, according to embodiments of the invention.

Fig. 11 shows performance of random shuffling compared with bootstrap resampling, according to an embodiment of the invention.

Fig. 12 shows examples of resampling distributions of COPA scores for datasets, according to embodiments of the invention.

Fig. 13 shows fitting of a resampling with function towards the tail of the empirical distribution, according to an embodiment of the invention.

Fig. 14 shows a match between fitted p-values, according to embodiments of the invention.

Fig. 15 shows performance of binomial resampling and bootstrap + function compared to the bootstrap resampled p-values, according to embodiments of the invention.

Fig. 16 shows performance of binomial resampling and bootstrap + function compared to the bootstrap resampled p-values, according to embodiments of the invention.

Fig. 17 shows performance of bootstrap resampling or bootstrap + function compared to binomial, according to embodiments of the invention.

Fig. 18 shows binomial compared to the bootstrap resampling, according to embodiments of the invention.

Fig. 19A shows a gene profile with corresponding scoring information, according to embodiments of the invention.

Fig. 19B shows a waterfall plot of a gene expression in one of five studies, according to an embodiment of the invention.

Fig. 19C shows a gene expression (2log of fold change, y-axis) plotted against Copy Number Variation (x-axis), according to an embodiment of the invention.

Fig. 19D shows meta-analysis of five studies that identifies one gene as most significant outlier gene (using product rank), according to an embodiment of the invention.

Fig. 19E shows a heatmap showing the normalized rank score for each gene across all studies of the meta analysis, with log 10 of the Q-value, according to an embodiment of the invention.

Fig. 19F shows survival analysis in TCGA shows that outlier subjects with >7 fold upregulation of FAM5C vs those with no major fold change (<2), have poorer Overall Survival, according to embodiments of the invention.

Fig. 19G shows BaseSpace Correlation Engine Disease Atlas app showing that there are 31 public Breast cancer studies in which FAM5C is also up-regulated, according to an embodiment of the invention.

Fig. 20 shows an illustration of the binomial method, according to an embodiment of the invention.

## DETAILED DESCRIPTION

Reference will now be made in detail to the disclosed embodiments, examples of which are illustrated in the accompanying drawings. Wherever convenient, the same reference numbers will be used throughout the drawings to refer to the same or like parts.

Some embodiments of the current invention are discussed in detail below. In describing embodiments, specific terminology is employed for the sake of clarity. However, the invention is not intended to be limited to the specific terminology so selected.

Analysis of gene expression is a powerful method to discover genes driving cancer and other diseases, yet most methods are only sensitive to those genes that are dysregulated in the majority of subjects. Although in many cases driver genes are only dysregulated in smaller subsets of subjects. For example, the discovery of HER2 (ERBB2) overexpression in a subset of about 20% breast cancer subjects led to the development of successful targeted therapies such as Trastuzumab. Discovering deregulated disease genes as biomarkers and drug targets is a major focus of academic and pharmaceutical research. Within Oncology, but potentially also in the study of other diseases or healthy populations, small subsets of subjects may display extreme outlier patterns (for example, but not limited to, the RNA expression of genes within a particular cancer type). Identifying genes with outlier patterns is of interest as they are likely to be involved in pathways that drive disease progression and may be effective drug targets. However, many of these are only deregulated in small groups of outliers within a disease, e.g. subjects with amplified and fusion genes in cancer. Common statistical tests that compare cases versus controls like a t-test, are not designed to find these outlier genes.

Recently, several methods were introduced to address this, including Cancer Outlier Profile Analysis (COPA), by Tomlins *et al.* COPA may determine which genes have heterogeneous expression within a collection of samples, such as samples collected from cancer patients. Exemplary COPA methods are described in "Recurrent fusion of TMPRSS2 and ETS transcription factor genes in prostate cancer" by Scott A. Tomlins, Daniel R. Rhodes, Sven Perner, Saravana M. Dhanasekaran, Rohit Mehra, Xiao-Wei Sun, Sooryanarayana Varambally, Xuhong Cao, Joelle Tchinda, Rainer Kuefer, Charles Lee, James E. Montie, Rajal B. Shah, Kenneth J. Pienta, Mark A. Rubin, and Arul M. Chinnaiyan. As will be described further below, COPA methods accentuate and identify outlier profiles by applying a numerical transformation based on the median and median absolute deviation of a gene expression profile in view of a quantile. However, COPA and other methods have several major shortcomings. One technical problem of applying COPA scores is that they are not directly comparable across different studies or with different input parameters. They do not provide a *p*-value but a score, and these scores are not comparable across studies for meta-analyses. Thus, current methods fail to assign significance to COPA scores (derived from COPA methods) within a single study or analysis, making it difficult to know which outlier genes identified in that analysis are actually statistically significant. Furthermore, the magnitudes of the COPA scores may not be directly comparable across different analysis, or under different input parameters. This may also limit integrating results across analyses in meta-analysis. Further, current solutions for meta-analyses of outlier scores, including rank-based methods and binomial test, also require large study numbers and do not adjust for study size differences.

Recently, the COPA approach has been extended to include a meta-analysis strategy, improving the power of detecting profound changes in expression by combining multiple studies. For example, Rhodes et al. [8] conducted a large-scale analysis of 31 gene expression profiling studies comprising nearly 3,200 microarray experiments and identified AGTR1, the angiotensin II receptor type I, to be markedly overexpressed in a subset of breast cancer subjects. One caveat of this meta-COPA study is that binomial test was adopted to perform the meta-analysis. Binomial test works by using the presence/absence of genes among the top 1% of genes with outlier expression profiles within each of multiple independent studies, but it actually uses only minimal information from the data. It is applicable when the number of studies is large; when the number of studies is small, ties are easily formed, leading to the lack of differentiation among top outlier genes.

Chang et al. [9] compared multiple methods for meta-analysis. Rank-based methods are generally robust and reliable. However, they treat each study equally and fail to account for the impact from different sample sizes of studies. The p-value based meta-analysis methods naturally adjust for the impact of sample sizes and allow comparison across different studies. Among those, Fisher's combined p-value method achieves the best performance in terms of statistically robustness and sensitivity in the method comparison [9], thus it is particularly valuable for meta-COPA analysis. Recently, the COPA approach was extended to include a meta-analysis strategy, improving the power of detecting profound changes in expression by combining multiple studies. For example, Rhodes *et al.* [8] conducted a large-scale analysis of 31 gene expression profiling studies comprising nearly 3,200 microarray experiments and identified AGTR1, the angiotensin II receptor type I, to be markedly overexpressed in a subset of breast cancer subjects. One caveat of this meta-COPA study is that binomial test was adopted to perform the meta-analysis. Binomial test works by using the presence/absence of genes among the top 1% of genes with outlier expression profiles within each of multiple independent studies, but it uses only minimal information from the data. It is applicable when the number of studies is large; when the number of studies is small, ties are easily formed, leading to the lack of differentiation among top outlier genes.

Chang *et al.* [9] compared multiple methods for meta-analysis. Rank-based methods are generally robust and reliable. However, they treat each study equally and fail to account for the impact from different sample sizes of studies. The p-value based meta-analysis methods naturally adjust for the impact of sample sizes and allow comparison across different studies. Among those, Fisher's combined p-value method achieves the best performance in terms of statistically robustness and sensitivity in the method comparison [9], thus it is particularly valuable for meta-COPA analysis.

The systems and methods disclosed herein address certain shortcomings of the art and/or to develop statistical methods to assess significance of COPA scores. According to certain disclosed systems and methods, embodiments can compute p-values for COPA scores. For example, aspects of the disclosure can include Bootstrap resampling, Bootstrap with function (e.g., generalized Pareto distribution (GPD)) and a Binomial resampling method for statistical significance of COPA (ssCOPA). With bootstrap resampling, its precision can be limited by the number of randomizations performed. To reduce the number of randomizations needed, we applied tail approximation from generalized Pareto distribution (GPD) to the distribution of bootstrapped values. As both methods are still computational intensive, we developed a non-parametric solution to compute p-values via Binomial distribution. We describe each aspect.

Performance of some aspects can be seen using breast cancer studies. In some embodiments, binomial resampling p-values match those of Bootstrap resampling using 10,000 randomizations, while requiring less compute than Bootstrap resampling or Bootstrap with function. Embodiments of the current invention having *p*-value-based approaches can achieve significantly better concordance between the small and large meta-analysis (*p*-value= $10^{-17}$~$10^{-24}$), than traditional COPA with Binomial test (*p*-value= $10^{-14}$). Binomial resampling can allow a more sensitive and faster identification of important driver genes in cancers and other diseases and is available in GitHub and we have implemented some improvements to COPA in the BaseSpace™ Cohort Analyzer platform from Illumina (formerly NextBio Clinical).

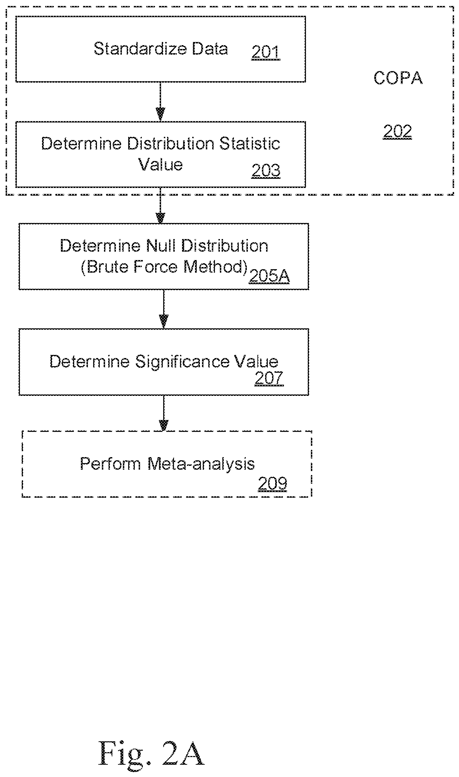

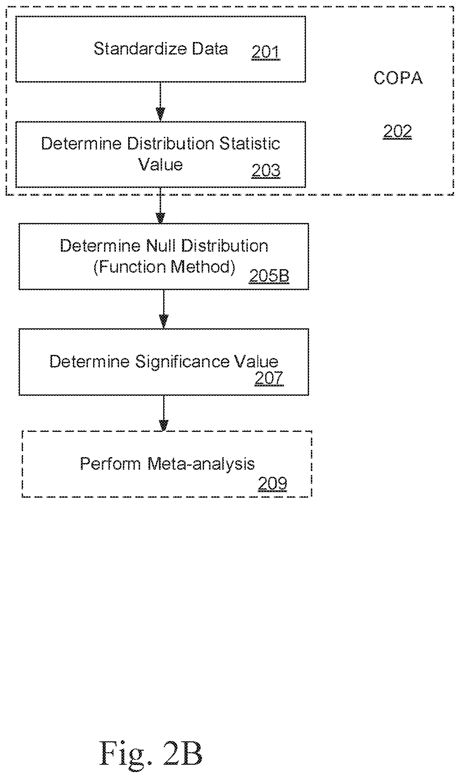

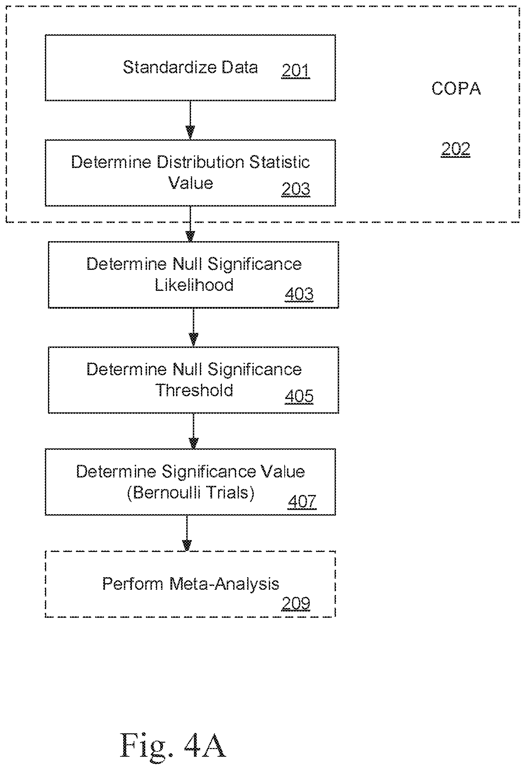

COPA scores may be determined for each factor within an analysis. Factors may be any subset of genomic material (such as, but not limited to a gene, region of a gene or subset of genes) for which continuous-valued observations (such as but not limited to integer and non-integer values reflective of RNA expression, protein expression, miRNA expression, other clinical measurements such as cholesterol values, or any other type of genomic expression observations) may be associated. Significance values (such as, but not limited to a p-value) may be computed for each COPA score within the analysis. Meta-analysis, based on the significance values, may be performed across multiple analyses that include at least some of the same factors to yield revised significance values for the factors in the meta-analysis. This is discussed in further detail below at least in connection with Fig. 2A, Fig. 2B, and Fig. 4A below.

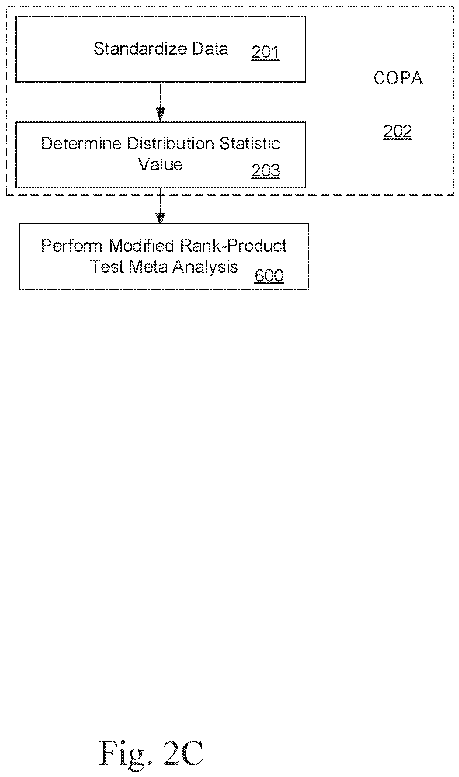

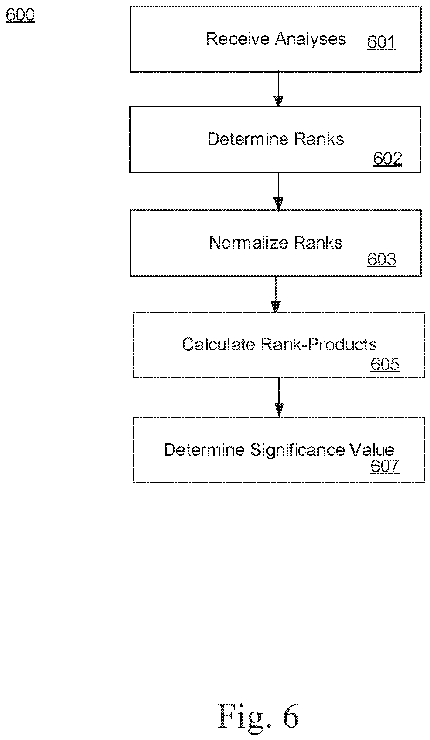

In some aspects, meta-analysis with factors ranked based on COPA scores may be performed to determine significance values for factors. This may be performed with or without determining significance values in each individual analysis. This is discussed in further detail below at least in connection with Fig. 2C, and Fig. 6 below.

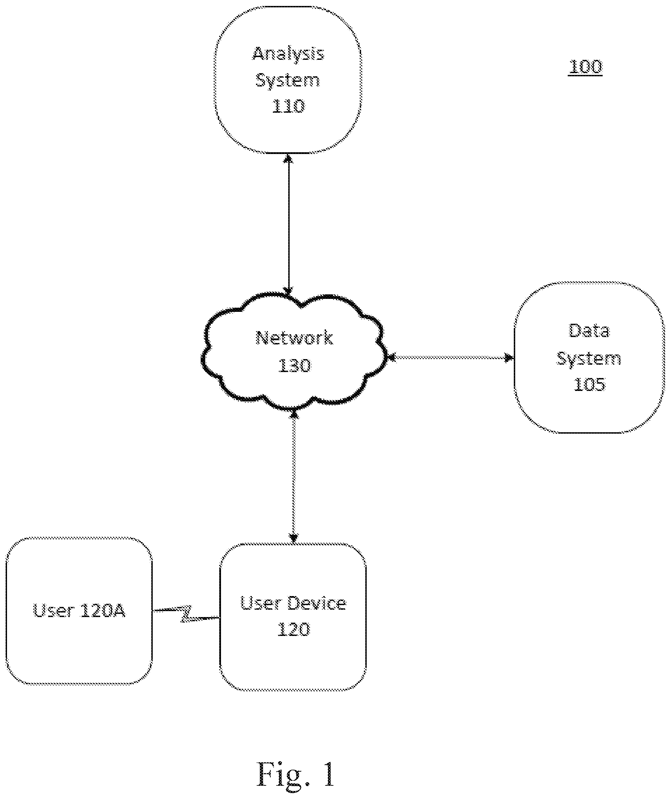

Fig. 1 depicts an exemplary schematic of a system for determining the significance of outliers in observational data. System 100 may comprise a data system 105, an analysis system 110, and a user device 120 configured to communicate over network 130. Consistent with disclosed embodiments, user 120A may interact with system 100 to determine the significance of factors corresponding to continuous-valued observations in observational data.

Data system 105 may comprise one or more systems for storing and extracting observational data. This observational data may be continuous-valued observations corresponding to factors, as introduced above. In some aspects, the observational data may correspond to data from microarrays, such as DNA or RNA microarray data. In some aspects, the observational data may comprise gene expression information obtained from multiple samples of cells. As a non-limiting example, each sample in the observational data may comprise a continuous-valued observation, or measurement of the cellular transcriptome of a sample of cells, such as a sample of cells taken from a tumor. The value of the continuous-valued observational data may indicate a level of gene expression in the sampled cells. In some embodiments, the samples comprising the observational data may have been obtained from different patients. As a further non-limiting example, the observational data may indicate the presence and quantity of a specific sequence of genomic material, such as DNA or RNA, in a sample. In certain aspects, each factor may correspond to a specific sequence of genomic material, such as messenger RNA corresponding to a gene. In some embodiments, expression of certain RNA sequences may be identified as upregulated or downregulated in certain tissues. A non-limiting example of such tissues includes tumors.

As introduced above, and discussed in certain embodiments below, the observational data stored in data system 105 may be values (also termed as continuous-valued observations) indicative of a level of expression of a factor, such as a gene or other subset of genomic material. A COPA method may be applied to this observational data to yield a distribution statistic value, such as a COPA score, for the factor. Then, a significance value indicative of statistical significance can be determined for the COPA score. This statistical significance may be used to evaluate whether an identified factor warrants further investigation.

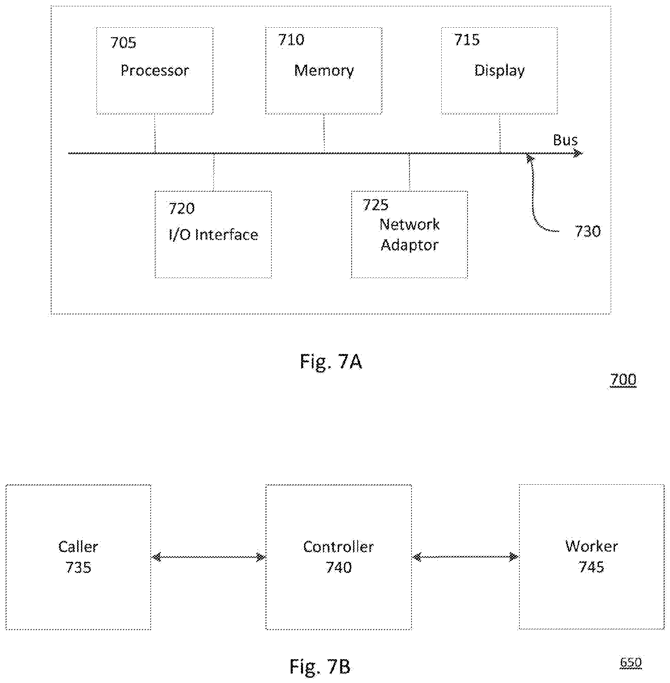

Analysis system 110 may comprise one or more computing systems. An exemplary component of analysis system 110 is described below with respect to Fig. 7A. In some embodiments, analysis system 110 may comprise a parallel computing environment. In some aspects, this parallel computing environment may be implemented according to the MapReduce architecture described in "MapReduce: Simplified Data Processing on Large Clusters," by Jeffrey Dean and Sanjay Ghemawat.

In various aspects, this parallel computing environment may be implemented according to the Spark architecture described in "Spark: Cluster Computing with Working Sets," by Matei Zaharia, Mosharaf Chowdhury, Michael J. Franklin, Scott Shenker, and Ion Stoica. As would be recognized by one of skill in the art, these implementations are intended to be exemplary, and not limiting. In various embodiments, analysis system 110 may be implemented using one or more computing clusters, servers, workstations, desktops, or laptops. In some embodiments, analysis system 110 may be implemented using a cloud-based computing environment. In some embodiments, analysis system 110 may be configured to analyze data. The data may comprise observational data, which may be received from one or more other components of system 100, such as data system 105 or user device 120, or another system.

User device 120 may comprise a computing system configured to communicate with the other components of system 100. An exemplary component of user device 120 is described below with respect to Fig. 7A. User device 120 may be configured to send or receive data or instructions from at least one of analysis system 110 and data system 105. User device 120 may be configured to communicate with the other components of system 100 over network 130. In some embodiments, user device 120 may operate as a client of another component of system 100. User device 120 may include, but is not limited to, one or more servers, workstations, desktops, or mobile computing devices (e.g., laptops, tablets, phablets, or smart phones). User device 120 may be configured to enable interaction with user 120A. In some aspects, user device 120 may provide a graphical user interface for displaying information. The displayed information may be received by user device 120, or may be generated by user device 120. As a non-limiting example, the displayed information may include at least a portion of the observational data or results generated by system 100 (e.g. significance values).

Consistent with disclosed embodiments, user 120A may interact with user device 120 to use system 100. In some embodiments, user 120A may interact with user device 120 to cause data system 105 to provide data. The data provided may include observational data. The data provided may include other analyses or datasets, rankings, significance values, or other information. The provided observational data may have been generated by data system 105, by another component of system 100, or by another system. The data may be provided to analysis system 110, or to another component of system 100. In certain embodiments, user 120A may interact with user device 120 to cause analysis system 110 to determine significance values. In some embodiments, the significance values describe the significance of factors corresponding to observations (such as continuous-valued observations) in the observational data. In some embodiments, user 120A may interact with user device 120 to cause analysis system 110 to perform a meta-analysis, as described below. In some aspects, user 120A may interact with user device 120 to cause analysis system 110 to provide data. The data provided may include significance values. The provided significance values may have been generated by analysis system 110, by another component of system 100, or by another system. The data may be provided to user device 120, or to another component of system 100, or to another system.

Network 130 may be configured to provide communications between components of Fig. 1. As a non-limiting example, network 130 may be any type of network (including infrastructure) that provides communications, exchanges information, and/or facilitates the exchange of information, such as the Internet, a Local Area Network, or other suitable connection(s) that enables system 100 to send and receive information between the components of system 100, between the components of system 100 and other systems, and between system 100 and other systems.

Fig. 2A, Fig. 2B, and Fig. 2C depict exemplary flowcharts of methods for determining the significance of factors describing observational data. As illustrated in Fig. 2A, Fig. 2B, and Fig. 2C, continuous-valued observations corresponding to factors may be standardized (as discussed in connection with step 201). From the standardized continuous-valued observations, a distribution statistic value may be determined (as discussed in connection with step 203) for each factor. Optionally, in certain embodiments, steps 201 and 203 may be performed as part of a COPA method 202, as indicated with the dotted lined box. When performed as part of a COPA method 202, the distributions statistic value may be a COPA score. In contrast, when a COPA method 202 is not performed, the distribution statistic value may be a distribution statistic value derived from any other distribution statistic, such as (but not limited to) a mean, median, mode, Outlier Sum (as described in further detail in "Outlier sums for differential gene expression analysis" by Robert Tibshirani and Trevor Hastie) or Maximum Ordered Subset t-statistic (as described in further detail in "MOST: detecting cancer differential gene expression" by Heng Lian).

As illustrated in Fig. 2A and Fig. 2B, from the distributions statistic value, a null distribution may be determined by either a function method (as discussed in connection with step 205B) or a bootstrap resampling method (as discussed in connection with step 205A). A significance value may be calculated (as discussed in connection with step 207) using the null distribution. Optionally, the significance value may be used with other analyses to perform meta-analysis (as discussed in connection with optional step 209 via dotted lines) to yield revised significance values.

Observational data on which the methods of Fig. 2A, Fig. 2B, and Fig. 2C operate may comprise data stored in at least one non-transitory memory. In some embodiments, the observational data may comprise a plurality of samples of an analysis. Each of the samples may comprise a plurality of continuous-valued observations. Each of the continuous-valued observations may correspond to a factor. As a non-limiting example, the observational data may be stored, organized, or provided in the form of a table, or matrix with columns corresponding to samples, rows corresponding to factors, and entries corresponding to continuous-valued observations.

In reference to Fig. 1, in some aspects, analysis system 110 may be configured to receive observational data from samples to perform the methods described in connection with Fig. 2A, Fig. 2B, and Fig. 2C. As a non-limiting example, analysis system 110 may be configured to receive observational data comprising samples from one or more other elements of system 100, or another system. As a non-limiting example, analysis system 110 may be configured to receive the observational data comprising samples from data system 105. As an additional non-limiting example, analysis system 110 may be configured to receive the observational data comprising samples from user device 120. In various aspects, receiving the observational data comprising samples may comprise retrieving the observational data comprising samples from a non-transitory memory, for example a non-transitory memory associated with an element of system 100, or another system.

Returning to Fig. 2A, Fig. 2B, and Fig. 2C, in step 201, analysis system 110 may be configured to standardize the observational data, consistent with disclosed embodiments. In some aspects, the values of the continuous-valued observations may not be identically distributed by factor. As a non-limiting example, the values of continuous-valued observations for a first factor may be drawn from a different probability distribution than the values of continuous-valued observations for a second factor. Analysis system 110 may be configured to standardize the observational data, causing the continuous-valued observations to approximate samples drawn from identically distributed, independent random variables. This standardization may enable the disclosed systems and methods to better estimate a null distribution for each factor.

For example, in step 201, analysis system 110 may be configured to determine measures of central tendency and dispersion across the continuous-valued observations for a factor to standardize the observational data for that factor, consistent with disclosed embodiments. The measures of central tendency and dispersion may be determined for at least a portion of the factors in the observational data of an analysis. The values of the measures of central tendency and dispersion may comprise data stored in at least one non-transitory memory. In some embodiments, the measures of central tendency may comprise arithmetic means, geometric means, interquartile means or medians. In various embodiments, the measures of central tendency may comprise a robust estimator of location, as would be known to one of skill in the art. In some embodiments, the measures of dispersion may comprise standard deviations, interquartile ranges, median absolute deviations, mean absolute deviations, or maximum absolute deviations. In various embodiments, the measures of dispersion may comprise a robust estimator of scale, as would be known to one of skill in the art. As a non-limiting example, analysis system 110 may be configured to determine the median and median absolute deviation of the continuous-valued observations corresponding to a factor according to the formulas

$$M(\overline{X}) = \begin{cases} \overline{X}_{\left(\frac{n+1}{2}\right)}, \ n \text{ is odd} \\ \left( \overline{X}_{\left(\frac{n}{2}\right)} + \overline{X}_{\left(\frac{n}{2}+1\right)} \right)/2, \ n \text{ is even} \end{cases}$$

and $MAD(\overline{X}) = M(|\overline{X} - M(\overline{X})|)$, where $\overline{X}$ is the set of continuous-valued observations corresponding to the factor. Thus M is the median of the absolute values of the differences between the values of the observations in $\overline{X}$ and the median value of the observations in $\overline{X}$.

Further to step 201, analysis system 110 may be configured to use the measures of central tendency and dispersion to standardize the continuous-valued observations in the analysis. In some embodiments, analysis system 110 may be configured to scale the continuous-valued observations, consistent with disclosed embodiments. The scaled continuous-valued observations may be stored in at least one non-transitory memory. In some aspects, scaling the continuous-valued observations may include subtracting the continuous-valued observations corresponding to each factor by the value of the measure of central tendency for that factor then dividing by the value of the measure of dispersion for that factor.

In step 203, a value of a distribution statistic for a factor may be determined, consistent with disclosed embodiments. This value may comprise data stored in a non-transitory memory. In some embodiments, analysis system 110 may be configured to determine the value of the distribution statistic for the factor using the scaled continuous-valued observations corresponding to the factor. In some aspects, the distribution statistic may comprise a quantile or a percentile, such as the 95th or 5th percentile. As a non-limiting example, analysis system 110 may be configured to determine as the distribution statistic the 95th percentile value for the scaled, continuous-valued observations corresponding to the factor. In some embodiments, analysis system 110 may be configured to determine values of multiple distribution statistics for a factor. As a non-limiting example, analysis system 110 may be configured to determine both a 95th and a 5th percentile for a factor. In certain embodiments, the distribution static value may be a value determined from COPA as a COPA score.

Further to step 203, the distribution statistic may establish a threshold for the factor, indicating observations that are outliers due to being extreme with respect the remaining scaled continuous-valued observations for the factor. In certain embodiments, the value of the threshold may be used to determine a null significance threshold, as discussed further below in connection with Fig. 4A. As would be recognized by one of skill in the art, other distribution statistics may be used to establish a threshold. For example, the threshold may be established using the mean of the scaled, continuous-valued observations corresponding to the factor, plus some number of standard deviations.

Returning to Fig. 2A, Fig. 2B, and Fig. 2C, as indicated in the step 202 with dotted lines, steps 201 and 203 may be steps of a COPA method to determine a COPA score as the distribution statistic value for a factor. As a non-limiting example, as part of standardizing the continuous-valued observations discussed further in connection with step 201, the COPA method may include applying a numerical transformation to the continuous-valued observations of the factor based on the median and median absolute deviation. Then, as part of determining a value of a distribution statistic for the factor as discussed further in connection with step 203, a quantile may be taken of the transformed continuous-valued observation to produce a COPA score. The COPA score would correspond to the factor associated with the continuous-valued observations. Therefore, within an analysis, each factor may have multiple continuous-valued observations from different samples and a single COPA score derived from the multiple continuous-valued observations from the different samples.

In step 205A of Fig. 2A and step 205B of Fig. 2B, a null distribution of the distribution statistic may be determined, consistent with disclosed embodiments. The null distribution may comprise a statistical distribution of the distribution statistic under the assumption that the variance in the observational data depends on chance alone.

In certain embodiments, the null distribution may be determined empirically via a bootstrap method as illustrated in step 205A of Fig. 2A. For example, analysis system 110 may be configured to determine the null distribution for at least a portion of the factors or all the factors. In various aspects, analysis system 110 may be configured to determine the null distribution using a resampling technique. As a non-limiting example, analysis system 110 may be configured to determine the null distribution using bootstrapping. In some aspects, analysis system 110 may be configured to determine the null distribution using the standardized observational data. As a non-limiting example, analysis system 110 may be configured to randomly select a set of scaled continuous-valued observations from the standardized observational data to determine the null distribution. The number of observations in this set may equal the number of scaled continuous-valued observations in the standardized observational data corresponding to a factor. As a non-limiting example, when the observational dataset in an analyses is arranged as a matrix with rows corresponding to factors and columns corresponding to samples, the number of observations in this set may equal the number of columns in the observational dataset. Analysis system 110 may be configured to compute a value of the distribution statistic for the randomly selected set. Analysis system 110 may be configured to repeatedly and randomly select sets from the standardized observational data and calculate a value of the distribution statistic. This selection process may be performed with or without replacement. In some embodiments, analysis system 110 may be configured to repeat this process a predetermined number of times. As a non-limiting example, analysis system 110 may be configured to repeat this process 100 times, 1,000 times, or 1 million times. In certain embodiments, analysis system 110 may alternatively be configured to repeat this process until a predetermined amount of time has elapsed, such as until 10 seconds, 100 seconds, or 10,000 seconds. Analysis system 110 may be configured to use the values of the distribution statistic for the randomly selected sets to determine significance values.

In some embodiments, analysis system 110 may be configured to determine the null distribution via the function method, as discussed in connection with 205B of FIG. 2B, without completing a bootstrap resampling method determination of the null distribution described above in connection with step 205A, consistent with disclosed embodiments. In some embodiments, as described above, analysis system 110 may be configured to generate bootstrap values. In certain aspects, analysis system 110 may be configured to estimate a portion of the null distribution using the bootstrap values (and not complete a bootstrap method determination of the null distribution). As a non-limiting example, a tail of the null distribution may be estimated by analysis system 110. This tail may be an upper tail or a lower tail of the null distribution. In some aspects, analysis system 110 may be configured to estimate the tail of the null distribution by fitting a function to at least a portion of the bootstrapped values. Analysis system 110 may be configured to accomplish this function fitting using methods and systems known to one of skill in the art. As a non-limiting example, analysis system 110 may be configured to select function parameters that minimize some functional, such as a mean-squared error, over a range. The range may be a portion of the null distribution, such as the tail of the null distribution. The function may be a normal distribution or a "heavy-tailed" distribution, consistent with disclosed embodiments. In some embodiments, the function may be a power-law distribution. As a non-limiting example, the function may be Pareto distribution, and may be described by a scale parameter $x_m$ and a shape parameter $\alpha$.

Further to step 205B, in some embodiments, analysis system 110 may be configured to use a reduced number of bootstrap samplings when estimating the tail of the null distribution with a function (as part of the function method) in contrast with the number of bootstrap samplings when performing the bootstrap resampling method as discussed in connection with step 205A of Fig. 2A. As a non-limiting example, in performing the function method as discussed in connection with step 205B, analysis system 110 may be configured to randomize 50 times, 75 times, or 100 times, to generate the null distribution when estimating the tail of the null distribution with a function. As would be appreciated by one of skill in the art, this reduction in the number of randomizations improves the efficiency, and reduces the memory and processing requirements, of system 100. Thus these embodiments constitute an improvement to a technology and a technical field, and an improvement to the functioning of system 100 itself.

In step 207 of Fig. 2A and Fig. 2B, the null distribution may be used in part to determine significance values, consistent with disclosed embodiments. The significance values may comprise data stored in at least one non-transitory memory. In some embodiments, analysis system 110 may be configured to determine at least a portion of the significance values based on the null distribution. As a non-limiting example, in some aspects, analysis system 110 may be configured to determine a significance value for a factor using values randomly generated according to the null hypothesis of the null distribution (determined via either the bootstrap resampling method discussed in connection with step 205A of Fig. 2A or the function method discussed in connection with step 205B of Fig. 2B). Further to the exemplary embodiment including the COPA method 202 discussed above, each factor may have multiple continuous-valued observations from different samples, a single COPA score derived from the multiple continuous-valued observations from different samples, and a significance value (such as a p-value) based on the COPA score. As a non-limiting example, analysis system 110 may be configured to determine a significance value for a factor as a proportion of randomly generated values exceeding the value of the distribution statistic for the factor. The randomly generated values exceeding the value of the distribution statistic for the factor may be found as part of the null distribution. As a non-limiting example, the $90^{th}$ percentile (as an example quantile as discussed in connection with step 203) score for a factor may be 30.

Further to step 207, as described above, the null distribution may be estimated by repeatedly generating $90^{th}$ percentile scores for randomizations of the observational data. In this non-limiting example, 5 of 10,000 randomizations generated in this fashion may exceed the value 30 when performing the bootstrap resampling method discussed in connection with step 205A of Fig. 2A. Therefore, in this non-limiting example, the significance value for the factor may be 5 over 10,000, or 0.0005.

Further to step 207, in some embodiments, analysis system 110 may be configured to determine a significance value for a factor using the function determined as part of the function method discussed in connection with step 205B of Fig. 2B. In some aspects, analysis system 110 may be configured to determine the significance value by estimating an area under the function. In certain aspects, the value of this area may be the significance value. In various embodiments, analysis system 110 may be configured to determine the significance value using a cumulative distribution function corresponding to the estimated function. As a non-limiting example, when the estimated function is a generalized Pareto distribution with a scale parameter $x_m$ and a shape parameter $\alpha$, then the cumulative distribution function may be found

$$P(X \leq x) = \begin{cases} 1 - (x_m/x)^{\alpha}, & x \geq x_m \\ 0, & x < x_m \end{cases}$$

as . Analysis system 110 may be configured to directly determine the significance of the factor by substituting into the cumulative distribution function the value of the distribution statistic for the factor. This determination may depend on whether the factor has a value lying on the upper or lower tail of the null distribution. In a non-limiting example, supposing $x_m = 5$, $\alpha = 2$, and the value of the $90^{th}$ percentile score for a factor is 30, then the probability of a $90^{th}$ percentile score less than 30 is $P(X \leq 30) = 0.97$. Analysis system 110 may be configured to estimate the significance of the factor as one minus the value of the cumulative distribution function, or 0.03 in this non-limiting example. As an additional non-limiting example, supposing $x_m = 6$, $\alpha = 2$, and the value of the $10^{th}$ percentile score for a factor is 6.1, then the probability of a $10^{th}$ percentile score less than 6.1 is $P(X \leq 6.1) = 0.03$. Analysis system 110 may be configured to estimate the significance of the factor as the value of the cumulative distribution function, or 0.03 in this non-limiting example.

Further to step 207, analysis system 110 may be configured to output significance values for the factors, consistent with disclosed embodiments. In some embodiments, as described above, the significance values may be based on the value of the distribution statistic and the null distribution. In various embodiments, outputting the significance values may comprise at least one of displaying and/or printing, storing, or providing at least a portion of the significance values by analysis system 110. In certain aspects, analysis system 110 may be configured to store at least a portion of the significance values in a non-transitory memory. In various aspects, analysis system 110 may be configured to provide the significance values to one or more other components of system 100, or to another system. As a non-limiting example, analysis system 110 may be configured to provide at least a portion of the significance values to user device 120. User device 120 may be configured to perform at least one of displaying and/or printing, storing, or providing at least a portion of the significance values. As would be recognized by one of skill in the art, displaying and printing may encompass a range of visual presentation methodologies, and the disclosed subject matter is not intended to be limited to a particular method.

Optionally, as indicated with the dotted line in Fig. 2A and Fig. 2B, certain embodiments may perform optional step 209 to generate revised significance values through a meta-analysis, consistent with disclosed embodiments. In some embodiments, this meta-analysis may combine analyses. Analysis system 110 may be configured to receive or generate (using the methods described herein and/or other methods not described herein) the additional analyses included in this meta-analysis. As a non-limiting example, analysis system 110 may be configured to receive the at least some of the analyses from another component of system 100, or another system. As an additional example, analysis system 110 may be configured to generate at least some of the analyses from observational data received from another component of system 100, or another system. Such a meta-analysis may enable users to combine results for multiple sets of observational data to determine the most significant factors across all sets of observational data.

Further to step 209, in some embodiments, analysis system 110 may be configured to use Fisher's combined probability test to determine revised significance values (such as but not limited to revised p-values) for each factor across analyses. As a non-limiting example, analysis system 110 may be configured to use Fisher's combined probability test to determine revised significance values, for each factor based on significance values from one analysis and significance values from a second analysis. In various embodiments, analysis system 110 may be configured to use another type of meta-analysis (such as, but not limited to a rank-product test) to determine revised significance values for each factor.

With reference to Fig. 2C, in some embodiments, analysis system 110 may be configured to use a modified rank-product test to determine significance values for each factor based on the analyses. In some embodiments, analysis system 110 may be configured to use the rank-product test in the absence of significance values. As illustrated in Fig. 2C, a modified rank-product type of meta-analysis (illustrated in FIG. 2C as step 600 and discussed further in connection with Fig. 6) may be performed using distribution statistics produced in step 203 in the absence of determining significance values as discussed in connection to step 207 in Fig. 2A and 2B. As will be discussed further below, exemplary rank-product tests are described in "The exact probability distribution of the rank product statistics for replicated experiments," by Rob Eisinga, Rainer Breitling, and Tom Heskes, and "A fast algorithm for determining bounds and accurate approximate p-values of the rank product statistic for replicate experiments," by Rob Eisinga, Rainer Breitling, and Tom Heskes.

The modified rank-product method utilizes normalizing ranks in order to account for missing values (ranks) across analyses. Accounting for missing values across analyses is not covered in the original methodology of the Eisinga et al. references. As will be discussed in further detail below, the normalizing ranks is performed by calculating a scaling factor for each analysis and multiplying a rank for each factor in an analysis by the scaling factor. The scaling factor for an analysis may depend on a number of factors in an analysis, and may depend on a size of the complete set of factors across all analysis in the modified rank-product meta-analysis. The scaling factor for an analysis may be the ratio of the size of the complete set of factors across all analysis to the number of factors in the analysis.

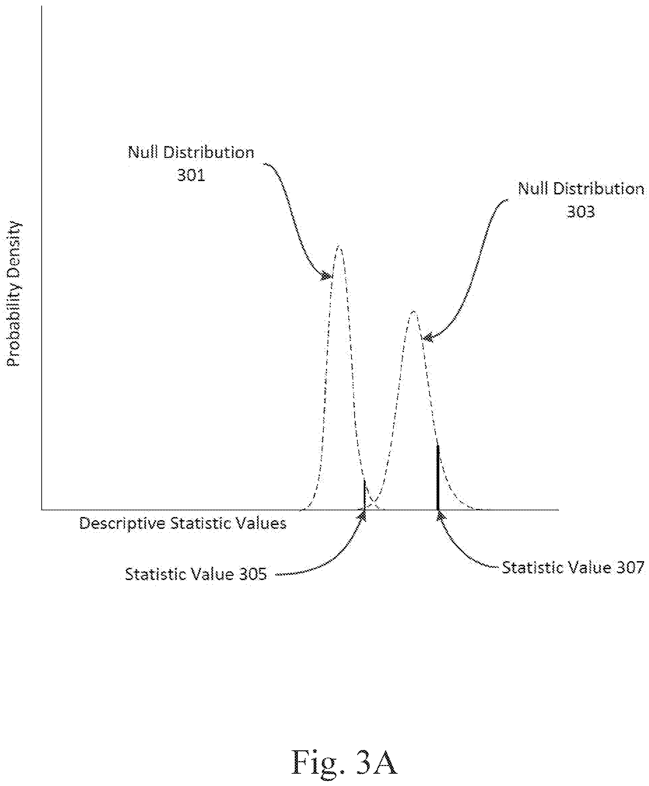

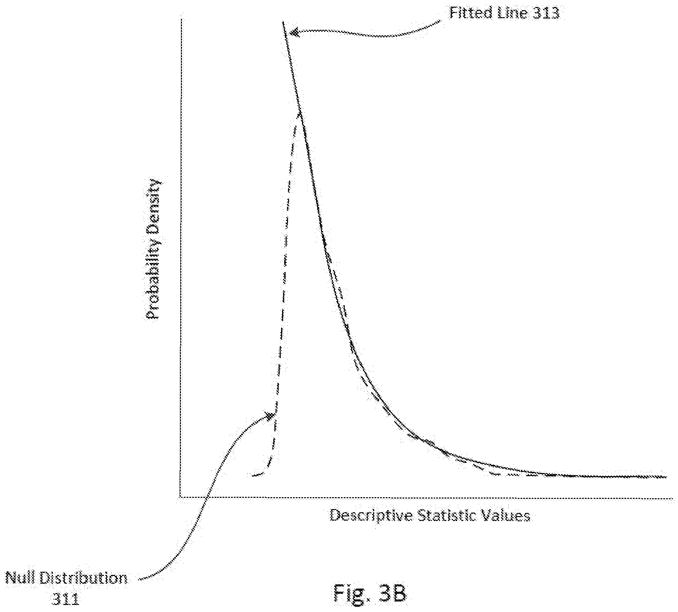

Figs. 3A and 3B depict exemplary null distributions estimated according to the envisioned systems and methods. These null distributions were calculated using the Cancer Genome Atlas Breast Invasive Carcinoma (TCGA BRCA) analysis, standardized using the median and median absolute deviation as described above with regards to Figs. 2A, 2B, and 2C. Fig. 3A depicts two exemplary null distributions for two distribution statistics, consistent with disclosed embodiments. The depicted null distributions are probability density functions, as would be understood by one of skill in the art. For each depicted null distribution in Fig. 3A, the abscissa is a value for the descriptive statistic, and the ordinate is the corresponding probability density. In this non-limiting example, null distribution 301 may be the distribution of the 90th percentile for the standardized observational data, assuming that any variance in standardized observational data arises from chance. Likewise, null distribution 303 may be the distribution of the 95th percentile for the standardized observational data, assuming that any variance in standardized observational data arises from chance. In this non-limiting example, distribution statistic value 305 may be a value of the 90th percentile for the standardized observational data correspond to a factor. As shown in Fig. 3A, a region exists to the right of distribution statistic value 305 under the curve of null distribution 301. In some aspects, the area of this region may be the likelihood of a value of the 90th percentile for the standardized observational data correspond to a factor exceeding statistic value 305 due to chance. Likewise, distribution statistic value 307 may be a value of the 95th percentile for the standardized observational data correspond to a factor. As shown in Fig. 3A, a region exists to the right of distribution statistic value 307 under the curve of null distribution 303. In some aspects, the area of this region may be the likelihood of a value of the 95th percentile for the standardized observational data correspond to a factor exceeding distribution statistic value 307 due to chance. In some embodiments, as the descriptive statistic changes, the characteristics of the null distributions may change. In some aspects, at least one of the symmetry or extreme value behavior of the null distributions may change as the descriptive statistic changes. As a non-limiting example, as shown in Fig. 3A, when the descriptive statistic changes from the 90th percentile to the 95th percentile, the distribution becomes more heavy-tailed, with a greater likelihood of extreme values and increased positive skew. As would be recognized by one of skill in the art, the distributions of the lower side percentile (e.g., 5th and 10th percentile) may approximately mirror those of the higher side percentiles (e.g., 90th and 95th) depending on the skew of the standardized observational data.

Fig. 3B depicts an augmented exemplary null distribution for a distribution statistic in accordance with the disclosed embodiments. For the depicted null distribution in Fig. 3B, the abscissa is a value for the descriptive statistic, and the ordinate is the corresponding probability density. This non-limiting example depicts generation of an augmented null distribution for the 95th percentile. As described above with regard to Fig. 2, the continuous-valued observations in the analysis were standardized, and randomizations of the null distribution for the 95th percentile were generated. In this non-limiting example, 100 randomizations were generated. These randomizations describe null distribution 311. But because of the low number of randomizations, null distribution 311 exhibits poor behavior in the upper tail of the distribution. In this non-limiting example, a generalized Pareto distribution 313 is used to approximate the tail of null distribution 311. This substantially reduces the number of randomizations required to obtain precise estimates of significance values for unlikely events (i.e. those far along the tail of the distribution). Based on the distribution of randomizations, the generalized Pareto distribution 313 is fit to the tail of null distribution 311, as shown in Fig. 3B. The suitability of the function may be measured using a goodness-of-fit test. The fitted function may be used to directly estimate p-values (significance values) for extreme values of the descriptive statistic, as described above with regard to Fig. 2.

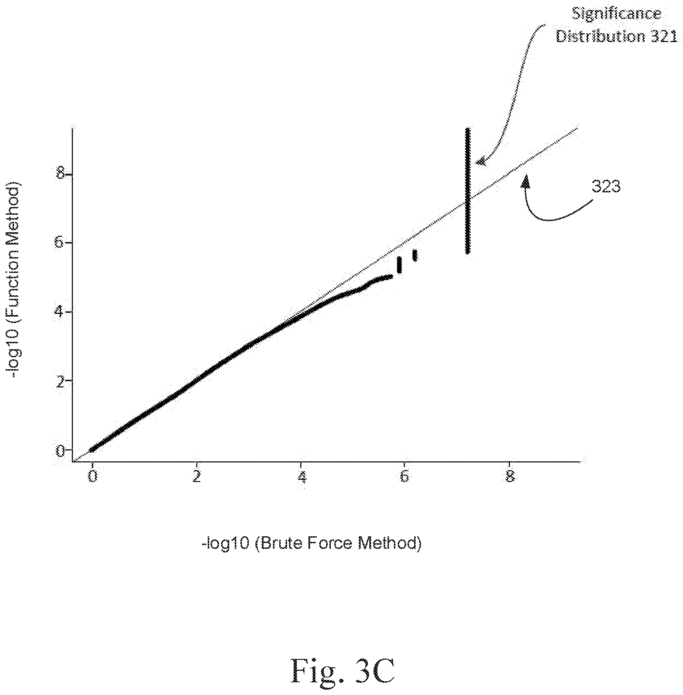

Fig. 3C depicts an exemplary comparison between two methods of estimating significance values, consistent with disclosed embodiments. This exemplary comparison also used the TCGA BRCA analysis, standardized using the median and median absolute deviation. The 95th percentile was calculated for each gene, i.e. factor, in the analysis. According to the bootstrap resampling method, the null distribution was estimated using 10,000 randomizations. The p-value (significance value) for each gene was calculated as the proportion of randomizations in the null distribution greater than or equal to the measured value of the 95th percentile for the gene. The abscissa for significance distribution 321 is the negative of the logarithm of the *p*-value for each gene, calculated according to the bootstrap resampling method. According to the function method, the null distribution was estimated using 100 randomizations. A generalized Pareto distribution was fitted to the tail of the 95th percentile distribution. The *p*-value for each gene was calculated based on the value of the 95th percentile for each gene and the cumulative distribution function of corresponding to the fitted generalized Pareto distribution. The ordinate for significance distribution 321 is the negative of the logarithm of the *p*-value for each gene, calculated according to this function method. When the *p*-values obtained by the bootstrap resampling method and function method for a gene are equal, the value of significance distribution 321 for the gene lies on the line 323. Where the significance distribution 321 lies below the line 323, the *p*-values obtained by the function method are less than the *p*-values obtained using the bootstrap resampling method. Many genes have the same value of the abscissa: for these genes, the same number of randomizations were greater than or equal to the measured value. Only by greatly increasing the number of randomizations could these ties be broken and accurate *p*-values for the observational data be obtained. In contrast, as may be observed from Fig. 3C, the function method tended to conservatively estimate *p*-values, but was able to estimate *p*-values for much more extreme values of the measured statistic using far fewer randomizations. As may be seen in Figs. 3A-3C, both the bootstrap resampling method and the function method represent improvements to a technology and a technical field, as they enable improved calculation of significance values for genes in observational analysis (e.g. the TCGA BRCA analysis) by system 100.

Fig. 4A depicts an exemplary flowchart of a calculation method for determining the significance of outliers in observational data, consistent with disclosed embodiments. According to the invention, this calculation method models estimation of the distribution statistic in terms of Bernoulli trials, allowing for an exact solution with minimal computations (by not requiring determination of a null distribution). As demonstrated above, determination of a null distribution contributes to a significant expenditure of computational resources. The function method may advantageously augment the null distribution as discussed above in connection with Fig. 2B. However, the calculation method provides even further advantages as there is no requirement to calculate a null distribution to produce a significance value. By providing an exact solution with minimal computation via the calculation method, the functioning of system 100 is improved by improving efficiency and reducing memory and processing requirements. Thus these embodiments constitute an improvement to a technology and a technical field, and an improvement to the functioning of system 100 itself.

As described above with regard to Fig. 1, analysis system 110 may be configured to receive observational data, consistent with disclosed embodiments. The observational data may comprise samples. The samples may include a number of continuous-valued observations corresponding to factors.

Also, as described above with regard to step 201 of Fig. 2A, Fig. 2B, and Fig. 2C, analysis system 110 may be configured to standardize the observational data in step 201 in Fig. 4A. In some embodiments, standardizing the data may comprise determining a measure of central tendency and dispersion. This measure of central tendency and dispersion may be determined for the continuous-valued observations corresponding to a factor. Various measures of central tendency and dispersion may be used, as described above with regard to step 201. As a non-limiting example, the measure of central tendency may be the median. As a non-limiting example, the measure of dispersion may be the median absolute deviation. In some embodiments, as described above with regard to step 201, analysis system 110 may be configured to scale the continuous-valued observations corresponding to the factor by the measure of central tendency and dispersion.

In step 203, analysis system 110 may be configured to determine values of the distributional statistic for the factors, as discussed above in connection Fig. 2A, Fig. 2B, and Fig. 2C. As a non-limiting example, analysis system 110 may be configured to determine a value of the distributional statistic for each factor corresponding to continuous-valued observations in the observational analysis. Values of the distributional statistic may comprise data stored in at least one non-transitory memory. In some aspects, the distributional statistic may comprise a quantile. As a non-limiting example, the distributional statistic comprising a quantile may comprise the 5th, 10th, 25th, 75th, 90th, or 95th percentile of continuous-valued observations corresponding to a factor. In various aspects, the distributional statistic may comprise a predetermined observation position in the ranked list of continuous-valued observations corresponding to a factor (e.g., the highest or lowest observation, the next highest or lowest observation, etc.).

As indicated in the step 202 with dotted lines as discussed further above in connection Fig. 2A-2C above, steps 201 and 203 may be steps of a COPA method to determine a COPA score as the distribution statistic value for a factor.

In step 403, analysis system 110 may be configured to determine a null significance likelihood, consistent with disclosed embodiments. Values of the null significance likelihood may comprise data stored in at least one non-transitory memory. In some embodiments, the null significance likelihood may depend on the standardized observational data. In certain aspects, analysis system 110 may be configured to determine the null significance likelihood for the factors. As a non-limiting example, analysis system 110 may be configured to determine a null significance likelihood for each factor corresponding to a subset of the continuous-valued observations in the observational analysis. In some embodiments, the null significance likelihood may depend on a value of the distributional statistic (distribution statistic value) determined in step 203.

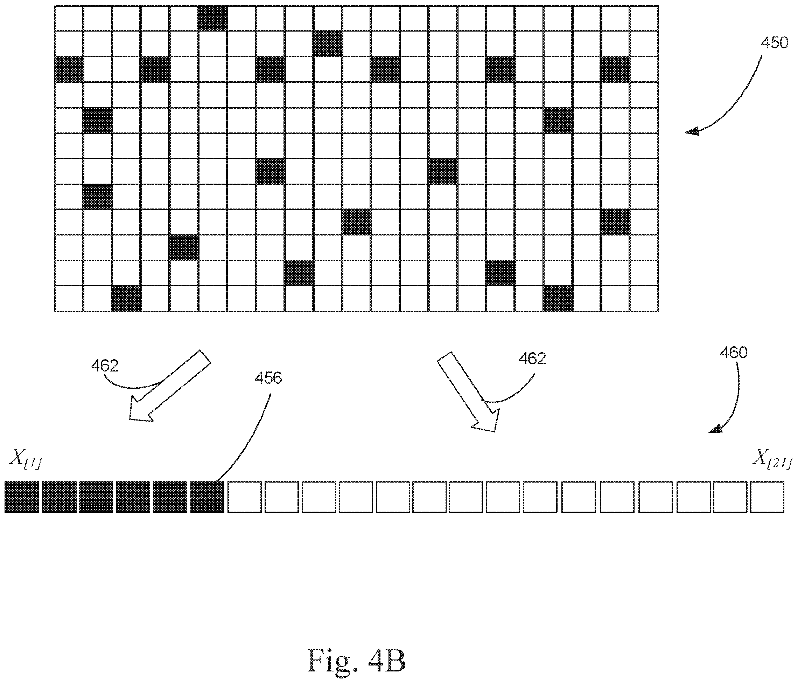

Further to step 403, in some embodiments, analysis system 110 may be configured to determine the null significance likelihood as the proportion of scaled continuous-valued observations in the standardized observational data more extreme than the value of the distributional statistic determined in step 203. Stated another way, this is the likelihood of selecting, in an analysis, a continuous-valued observation more extreme than the distribution statistic. When the distribution statistic concerns an upper quantile, such extreme data may have values equal to or greater than the value of the distributional statistic. When the distribution statistic concerns a lower quantile, such extreme data may have values equal to or less than the value of the distributional statistic. The null significance likelihood is discussed further below in connection with Figs. 4B.

In step 405, analysis system 110 may be configured to determine a null significance threshold for the factors, consistent with disclosed embodiments. As a non-limiting example, analysis system 110 may be configured to determine a value of the null significance threshold for the observational analysis depending on the value of the selected quantile and the number of samples within the observational data. Values of the null significance threshold may comprise data stored in at least one non-transitory memory. Analysis system 110 may be configured to determine a value of the null significance threshold depending on the selected quantile and the number of samples within the observational data.

Further to step 405, in some aspects, when the distribution statistic is a quantile, the distribution statistic may be located in a predetermined position in the ranked list of observational values corresponding to a factor. In some aspects, analysis system 110 may be configured to set this predetermined observation position as the null significance threshold. In various aspects, analysis system 110 may be configured to determine a null significance threshold based on the quantile.

Further to step 405, in some aspects, analysis system 110 may determine the null significance threshold using a formula for the estimation of this predetermined observation position for quantiles. In some aspects, this formula may depend on the number of continuous-valued observations associated with the factor and a quantile parameter. As a non-limiting example, the formula for determining this predetermined observation position may corresponding to a specific estimation type for quantiles. As a non-limiting example, the estimation type may correspond to the R-7 estimation type for quantiles in which the null significance threshold may depend on $h = (N - 1) \times q_i + 1$, where N is the number of continuous-valued observations associated with the factor, and $q_i$ is a quantile parameter. In some embodiments, analysis system 110 may be configured to set the null significance threshold as the nearest integer to $h$, the floor of $h$, or the ceiling of $h$. In some embodiments, a non-integer null significance threshold may be used to interpolate the approximate quantile using the incomplete beta function. The quantile parameter $q_i$ may be calculated as follows:

$$q_i = min(q, 1 - q)$$

, where $q$ is the quantile of the distribution statistic divided by the total number of quantiles. As a non-limiting example, when the distribution statistic is the 5th percentile and N equals 200, $q$ may be 0.05, $q_i$ may be 0.05, and $h$ may be 10.95. As an additional non-limiting example, where the distribution statistic is the 3rd quartile and N equals 200, $q$ may be 0.75 (i.e., third quartile divided by four quartiles), $q_i$ may be 0.25, and $h$ may be 50.75. In this manner, distribution statistics concerning both the upper and lower tails of the null distribution may be accommodated, consistent with disclosed embodiments.

Further to step 405, one of ordinary skill in the art would appreciate that the disclosed embodiments are not limited to these exemplary formulas. As apparent from these formulas, the null significance threshold may decrease as the quantile becomes more extreme. As a non-limiting example, when N is 200, the null significance threshold for the 10th percentile may be 20.9, and the null significance threshold for the 1st percentile may be 2.99. As an additional non-limiting example, when N is 200, the null significance threshold for the 80th percentile may be 40.8, and the null significance threshold for the 99th percentile may be 2.99.

In step 407, analysis system 110 may be configured to determine significance values for the factors in terms of Bernoulli trials, consistent with the invention. As discussed above in connection with Fig. 2A, analysis system 110 may be configured to determine a significance value for a factor as a proportion of randomly generated values exceeding the value of the distribution statistic for the factor. As a non-limiting example, analysis system 110 may be configured to determine a significance value for each factor corresponding to continuous-valued observations in an analysis. The significance values may comprise data stored in at least one non-transitory memory. In some embodiments, analysis system 110 may be configured to determine the significance value of the factor according to a distribution. In some aspects, this determination may use the null significance likelihood and the null significance threshold corresponding to a factor.

Further to step 407, as described above, analysis system 110 may be configured to model estimation of the distribution statistic in terms of Bernoulli trials. In such a trial, the likelihood of a value more extreme than the observed value is described by the cumulative binomial distribution:

$$\alpha = \sum_{i=\beta}^{N} \binom{N}{i} \rho^i (1-\rho)^{N-i}$$

, where $\alpha$ (the significance value) is the probability of a randomly sampling a value more extreme than the observed value, $N$ is the number of continuous-valued observations associated with the factor, $\beta$ is the null significance threshold, and $\rho$ is the null significance likelihood. In this fashion, analysis system 110 may be configured to determine the significance of a factor without estimating a null distribution through sampling (as discussed above in connection with Fig. 2A and Fig. 2B). Instead, the factor may be estimated directly as disclosed above.

Further to step 407, in such a trial, a randomly generated value of the distribution statistic may be more extreme than the observed value of the distribution statistic when a number of randomly selected continuous-valued observations corresponding to a factor (where the number of randomly selected continuous valued observations corresponds to the number of samples in an analysis) is at least equal to the null significance threshold are more extreme than the observed value of the distribution statistic. As a non-limiting example, if the predetermined observation position is the 3rd position, the distribution statistic is the value of ranked continuous-valued observations in the 3rd position (i.e, the value of the distribution statistic is the value of the third largest observation), then a randomly generated value of the distribution statistic may be more extreme than the observed value of the distribution statistic when at least three values randomly selected from the standardized observational data exceed the observed value. As a further non-limiting example, if the predetermined observation position is the first decile and the number of continuous-valued observations is 201, then a randomly generated value of the distribution statistic may be more extreme than the observed value of the distribution statistic when the observed value of the distribution statistic exceeds at least 21 values randomly selected from the standardized observational data.

Further to step 407, in some embodiments, analysis system 110 may be configured to use of the incomplete beta distribution in place of the cumulative binomial distribution:

$$\alpha = I_\rho(\beta, N - \beta + 1)$$

, where again $\alpha$ (the significance value) is the probability of a randomly sampling a value more extreme than the observed value, $N$ is the number of continuous-valued observations associated with the factor, $\beta$ is the null significance threshold, and $\rho$ is the null significance likelihood. This use of the incomplete beta distribution may enable analysis system 110 to use non-integer values of $\beta$ for the null significance threshold. Accordingly, analysis system 110 may be configured to use $h$ as the null significance threshold, rather than the nearest integer to $h$, the floor of $h$, or the ceiling of $h$.

Fig. 4B is an exemplary conceptual diagram of a standardized analysis 450 and a ranked list 460 of continuous-valued observations corresponding to a factor to elucidate the null significance likelihood and the null significance threshold, consistent with disclosed embodiments. The standardized analysis 450 is represented as a matrix with rows corresponding to factors and columns corresponding to samples. Standardization is discussed further above in connection with step 201 of Fig. 2A, Fig. 2B, Fig. 3C, and Fig. 4A. Each square represents a continuous-valued observation. Squares with solid fills represent a standardized continuous-valued observation that is more extreme than the value of an arbitrary distribution statistic.

Also illustrated in Fig. 4B are standardized continuous-valued observations for a factor from the analysis illustrated in Fig. 4B but with standardized continuous-valued observations that are reordered as a ranked list 460 for a randomized factor. This ranked list 460 for the randomized factor includes standardized continuous-valued observations randomly selected across all continuous valued observations in the analysis 450. Put another way, the ranked list 460 may correspond to a randomized factor in a bootstrap resampling of analysis 450. As part of the ranked list 460, these 21 standardized continuous-valued observations may be ordered such that each standardized continuous-valued observation is more extreme than the standardized continuous-valued observations to the right. Although there are 21 standardized continuous-valued observation in the illustrated embodiment, the number of standardized continuous-valued observations is arbitrary and chosen here for illustrative purposes but corresponds to the number of samples in the analysis 450. The first standardized continuous-valued observation in the ranked list may be termed as $X_{[1]}$ and the last may be termed as $X_{[21]}$. Squares with solid fills represent a standardized continuous-valued observation that are more extreme than the value of the distribution statistic.

Further to Fig. 4B, arrows 462 notes that the ranked list 460 is a collection of randomly selected continuous-valued observations from the analysis 450. The number of randomly selected continuous-valued observations corresponds to the number of samples in the analysis 450. The likelihood of a continuous-valued observation being a solid fill square is the null significance likelihood. Accordingly, the null significance likelihood is the proportion of solid filled squares over the total number of squares in analysis 450.

Also, further to Fig. 4B, the null significance threshold may be determined for a factor using Bernoulli trials without calculating the null distribution of the analysis 450. This may be based on an insight that the whole of the standardized values of an analysis, represented as Bernoulli trials, can be used to represent (and in place of determining) the null distribution for the analysis. Accordingly, in the ranked list 460, the solid filled square 456 is the standardized continuous-valued observation at the null significance threshold. Stated another way, the presence of the solid filled square 456 corresponding to the null significance threshold indicates that this ranked list 460 is as or more extreme than the distribution statistic. With reference to the R-7 estimation type for quantiles discussed above, the solid filled square 456 may be $X_{[6]}$, or $X_{[h]}$ where $h = (N - 1) \times q_i + 1$ (the null significance threshold), and where $h$ is 6, $N$ is 21 and $q_i$ is 1/4.

Returning to Fig. 4A, optionally, as indicated with the dotted lines, certain embodiments may perform step 209 to generate revised significance values through a meta-analysis, consistent with disclosed embodiments, as described above with respect to step 209 discussed further in connection to Fig. 2A and Fig. 2B. This meta-analysis may combine analyses, the analyses including the results determined in step 407. For example, as discussed above, in some embodiments, analysis system 110 may be configured to use Fisher's combined probability test to determine revised significance values for each factor based on significance values from a first analysis, such as determined in step 407, and significance values from a second analysis.

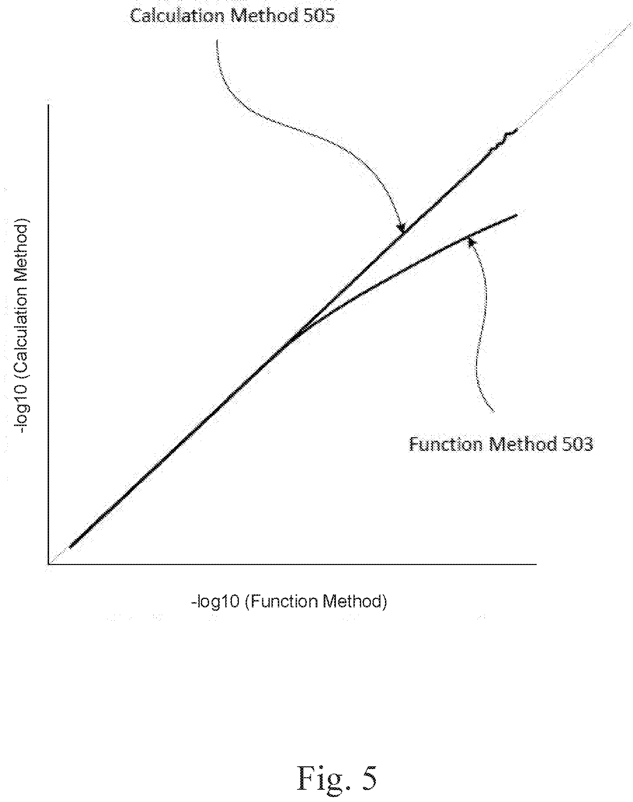

Fig. 5 depicts an exemplary comparison of the function method described with regard to bootstrap resampling in Fig 2B (function method 503) and the calculation method described with regard to Fig. 4A (calculation method 505). Significance values were calculated using each method for genes from five different breast cancer analyses within the Illumina® BaseSpace™ CohortAnalyzer platform. Significance values were also empirically determined for each gene from 10,000 samples of the null distribution as described with regard to Fig 2B. Inaccuracies in the calculation of significance values appear as deviations from the line of equality. As depicted in Fig. 5, calculated significance values using the calculation method 505 is much more accurate than significance values determined from approximating the null distribution using a generalized Pareto distribution in the function method 503. As shown, the calculation method 505 generally does not deviate from the line of equality, while the function method 503 increasingly underestimates the significance value as the empirically determined significance value decreases.

Fig. 6 depicts an exemplary method for performing a modified rank-product meta-analysis, consistent with disclosed embodiments. This method may include aggregating, or otherwise receiving analyses, as discussed further below in connection with step 601. An analysis may comprise data stored in at least one non-transitory memory. The received analyses may be ranked to produce ranked factors as discussed further below in connection with step 602. The received analyses may include differing numbers of factors, and the ranking of factors may differ between analyses. The received analyses may collectively define a complete set of factors, and each individual analysis may include at least some of this complete set of factors. The method may further include normalizing ranks to account for differing numbers of factors between analyses, as discussed further below in connection with step 603. As discussed further below in connection with step 603, ranks may be normalized by multiplying a rank for each factor in an analysis by a scaling factor that is the ratio of the number of the size of the complete set of factors across the analyses to the number of factors in the analysis. The method may additionally include calculating rank-products as discussed further below in connection with step 605. The method may additionally include determining significance values for factors using the rank-products as discussed further in connection with step 607. As discussed further below, the modified rank-product test method utilizes normalizing ranks in order to account for missing values across analyses, as not covered in the original methodology described in the Eisinga et al. references.

Analysis system 110 may be configured to receive analyses in step 601, consistent with disclosed embodiments. Analysis system 110 may be configured to receive analyses from one or more other components of system 100, such as data system 105, user device 120, or another system. Receiving multiple analysis may encompass retrieving analyses from a non-transitory memory, for example a non-transitory memory associated with an element of system 100, or another system. Receiving analyses may encompass generating at least one analysis, consistent with disclosed embodiments. In some aspects, analysis system 110 may be configured to generate at least one analysis using received observational data. This observational data may be received from one or more other components of system 100 (e.g., data system 105 or user device 1200, or another system. In some aspects, analysis system 110 may be configured to determine or receive scores for factors in the analyses, for example as described with regard to Fig. 2A, Fig. 2B, Fig. 2C, and Fig. 4A. As a non-limiting example, the scores for factors may be COPA scores as discussed further above.

In various aspects, analysis system 110 may be configured to rank factors in the observational data based on the scores in step 602. In various aspects, analysis system 110 may be configured to rank factors in the observational data based on the scores in step 602. As a non-limiting example, analysis system 110 may be configured to assign the lowest rank to the factor with the most extreme score, and successively higher ranks to factors with successively less extreme scores. Ties may be accounted for according to methods known to one of skill in the art. As another non-limiting example, analysis system 110 may be configured to assign the highest rank to the factor with the most extreme score, and successively lower ranks to factors with successively less extreme scores. As another non-limiting example, analysis system 110 may be configured to assign the lowest rank to the factor with the highest value of an upper quantile (e.g., the 90th or 95th percentile). As an additional non-limiting example, analysis system 110 may be configured to assign the lowest rank to the factor with the lower value of a lower quantile (e.g., the 1st or 2nd decile). With reference to the discussion of COPA scores in certain embodiments above, analysis system 110 may be configured to assign the lowest rank to the factor with the most extreme COPA score, and successively higher ranks to factors with successively less extreme COPA scores. Alternatively, analysis system 110 may be configured to assign the highest rank to the factor with the most extreme COPA score, and successively lower ranks to factors with successively less extreme COPA scores. Analysis system 110 may be configured to generate normalized ranks in step 603, consistent with disclosed embodiments. The normalized ranks may comprise data stored in at least one non-transitory memory. Analysis system 110 may be configured to determine normalized ranks for analyses received as described above. In some aspects, analysis system 110 may be configured to calculate a scaling factor for each analysis. The scaling factor for an analysis may depend on a number of factors in the analysis, and may depend on a size of the complete set of factors across all analyses in the modified rank-product test meta-analysis. The scaling factor for the analysis may be the ratio of number of the size of the complete set of factors across all analyses in the modified rank-product test meta-analysis to the number of factors in the analysis. Analysis system 110 may be configured to multiply the rank for each factor in the analysis by the scaling factor. As a non-limiting advantage of normalizing ranks in step 603, the modified rank-product test meta-analysis may perform meta-analysis among analyzes with different numbers of factors or different factors, rather than being constrained to perform meta-analysis among analyses with same factors as in the original rank-product test meta-analysis detailed in the Eisinga et al. references.

Further to step 603, as a non-limiting example, analysis system 110 may have received analyses defining a complete set of 20,000 factors across all the analyses in the modified rank-product test meta-analysis. The first analysis may include 18,000 of these 20,000 factors. Analysis system 110 may be configured to calculate the scaling factor for the first analysis as 20,000/18,000, or 1.11. Analysis system 110 may be configured to multiply the rank for each factor in the first analysis by 1.11. Thus, in the first analysis, the rank of first factor would be normalized to 1.11, the rank of the second factor would be normalized to 2.22, the rank of the third factor would be normalized to 3.33, etc.

Analysis system 110 may be configured to calculate rank-products in step 605, consistent with disclosed embodiments. Analysis system 110 may be configured to calculate a rank-product for a factor as the product of the normalized rank for the factor in each analysis of the analyses including the factor. As a non-limiting example, three of four analyses may include a factor. The normalized rank of the factor in the first analysis may be 19.5. The normalized rank of the factor in the second analysis may be 36.05. The normalized rank of the factor in the third analysis may be 14. The rank product for the factor may be 9841.65, the product of these three normalized ranks. Analysis system 110 may be configured to calculate the rank-product for at least some of the complete set of factors using the rank product, number of analyses including the factor, and size of the complete set of factors.

Analysis system 110 may be configured to determine significance values for factors in step 607, consistent with disclosed embodiments. Analysis system 110 may be configured to use normalized rank-products, such as those generated in step 605, as part of a rank-product test. Certain rank-product tests, such as the fast algorithm described by Eisinga et al. calculate significance values using a number of replications. In some embodiments, analysis system 110 may be configured to use the number of analyses including the factor as the number of replications. As a non-limiting example, when a factor appears in three of four analyses, the number of replications would be three.

Current solutions to performing meta-analysis, such as the ONCOMINE Binomial-Test meta-analysis procedure employed by the ONCOMINE product maintained by Thermo Fisher Scientific of Waltham Massachusetts, USA, may require a large number of analyses to be effective. The Binomial-Test meta-analysis procedure employed by the ONCOMINE is described in "ONCOMINE: A Cancer Microarray Database and Integrated Data-Mining Platform" by Daniel R. Rhodes, Jianjun Yu, K. Shanker, Nandan Deshpande, Radhika Varambally, Debashis Ghosh, Terrence Barrette, Akhilesh Pandey, and Arul M Chinnaiyan.

Advantageously, the disclosed systems and methods are able to identify outliers with better accuracy (greater sensitivity) than the ONCOMINE Binomial-Test meta-analysis at lower analysis count. Experiments were performed demonstrating that 5 analyses using either the bootstrap resampling method (discussed further below at least in connection with Fig. 2A), function method (discussed further below at least in connection with Fig. 2B), or calculation method (discussed further below at least in connection with Fig. 4A), along with the Fisher's combined probability test meta-analysis or the modified rank-product test meta-analysis (discussed further below at least in connection with Fig. 2C and Fig. 6) were able to identify outliers consistent to the ONCOMINE Binomial-Test meta-analysis on 31 analyses more effectively than the ONCOMINE Binomial-Test meta-analysis applied to the 5 analyses

The modified rank-product test may be preferable to the above discussed ONCOMINE Binomial-Test meta-analysis, as the modified rank-product test may be more sensitive at lower analysis count. This may be due to the modified rank-product test depending on the relative rankings of factors within an analysis, rather than treating similar ranking factors (such as similarly high ranking factors) within an analysis as equal as in the ONCOMINE Binomial-Test meta-analysis.

Example 1

Bootstrap resampling

To estimate the statistical significance, permutation is a standard practice when the sampling distribution is unknown [10]. However, this method cannot be employed in this situation, as permuting sample labels has no effect on the COPA score, which is obtained by ranking the values across samples of a gene and taking the specified percentile following standardization. As such we employ a slightly different strategy for randomization which we will refer to as Bootstrap resampling.

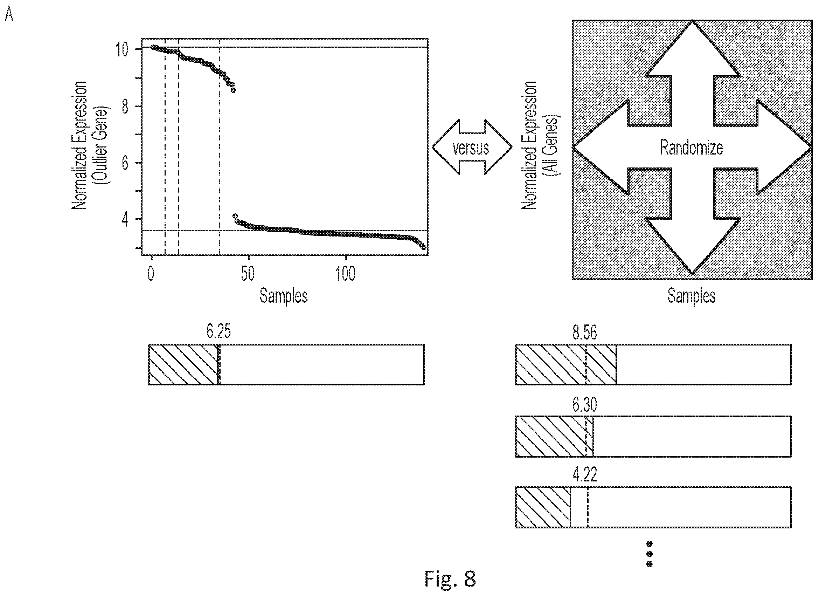

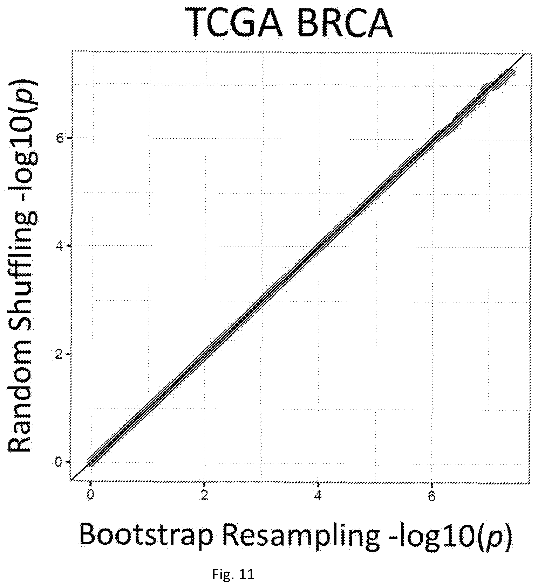

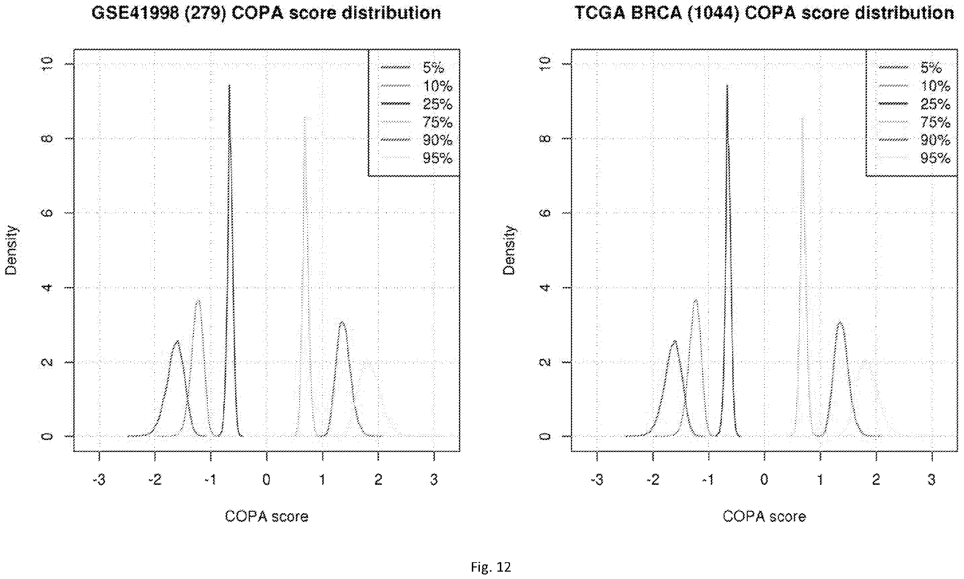

In order to perform a randomization test for COPA, the gene expression values need to be randomized across genes, however, expression values are not necessarily independently and identically distributed to each other. To solve this problem, the randomization is performed after standardization, which puts the values on the same scale. Thus, we can assume the standardized values are overall independently and identically distributed specific to the data type and platform (e.g. RNA-Seq - Illumina HiSeq). We perform resampling of the standardized values randomly with/without replacement (sampling with/without replacement does not affect results shown by Fig. 11) to produce a new matrix and take the percentile, i.e. COPA score, for each gene, shown in Fig 8. This resampling step is then repeated multiple times (e.g., 10,000) to generate a null distribution for COPA values. The p-value for the observed COPA scores can be easily obtained from this null distribution. Fig. 12 provides examples of resampling distributions of COPA scores for two breast cancer datasets (GSE41998 and TCGA breast cancer data-sets) at various percentiles. It is clear that the distribution changes from light tailed at 75% to heavy-tailed at 95%. And the distributions of COPA scores at lower-side percentiles (e.g. 5%, 10%, 25%) mirror those at upper-side percentiles (e.g., 95%, 90%, 75%).

One limitation of Bootstrap resampling is that resampled p-values are often capped by the number of randomizations performed, thus it is difficult to distinguish those top significant genes with true p-values exceeding the resolution of resampling.

Fig. 8 shows a schematic illustration of bootstrap resampling. The Bootstrap Resampling method approximates the significance of a COPA score through randomizations. Random draws can be obtained through the bootstrapping, random sampling with replacement, of the normalized expression matrix then calculating COPA scores for those randomizations. The red bar indicates sampled values that are as, or more, extreme than the observed COPA score. Note that a randomly sampled COPA score is more extreme than the observed score only when the number of sampled extreme values (red bar) exceeds the COPA threshold (dashed line). This can be a key observation that enables an exact calculation via binomial resampling.

Example 2

Bootstrap + generalized Pareto distribution (function method)

It is known that the resolution of resampling p-value is limited by the number of randomizations performed. To achieve higher resolution, it usually involves intensive computation, thus often infeasible for large-scale applications. Knijnenburg et al. [11] proposed to estimate the small permutation p-values using extreme value theory, i.e., by approximating tails of distributions with a generalized Pareto distribution. This can be extended to resampling p-values.

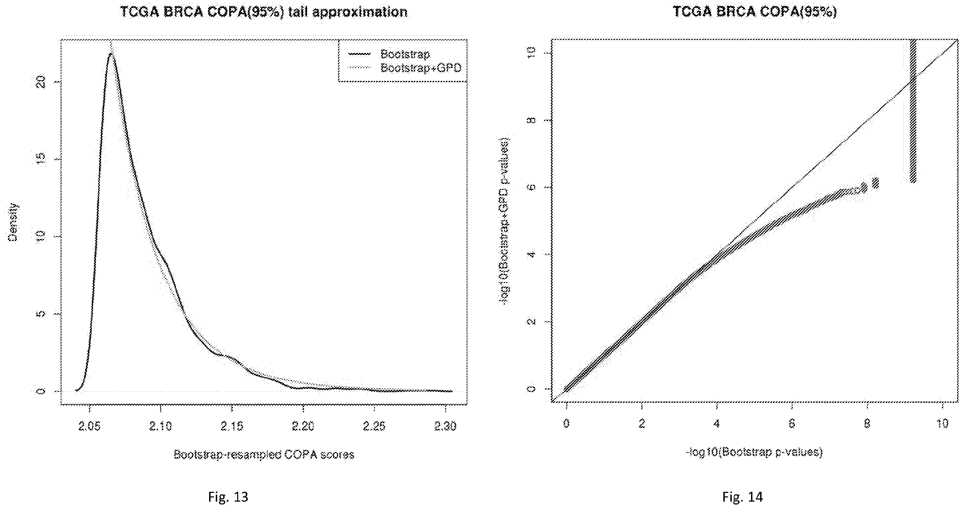

Bootstrap with function can thus be applied to TCGA BRCA data-set to approximate the tails of the null distribution of COPA scores generated by 100 randomizations from Bootstrap resampling. This substantially reduces the number of randomizations needed to produce p-values. Based on the distribution of resampled COPA scores, the upper (or lower as it is symmetric) tail (corresponding to 0.1% of the resampled COPA scores) can be fitted with the generalized Pareto distribution. The density plot (Fig 13) shows the fitting of GPD (orange line) towards the tail of the empirical distribution (black line). The fitting is reasonably good as verified by goodness-of-fit test (p-value = 1). Based on the fitted GPD, we can obtain the estimated p-values, which tend to be conservative as compared with resampled p-values. Fig 14 shows good match between fitted p-values and resampled p-values. When approaching the resolution limit of resampled p-values, appears the small deviation of fitted p-values from resampled p-values. The formation of vertical bar is because after exceeding the resolution limit, e.g 10-7, resampled p-values are identical for numerous top genes, while fitted p-values can still differentiate these genes. The limitation of resampling-related methods is still computational intensive, particularly infeasible for large-scale studies.

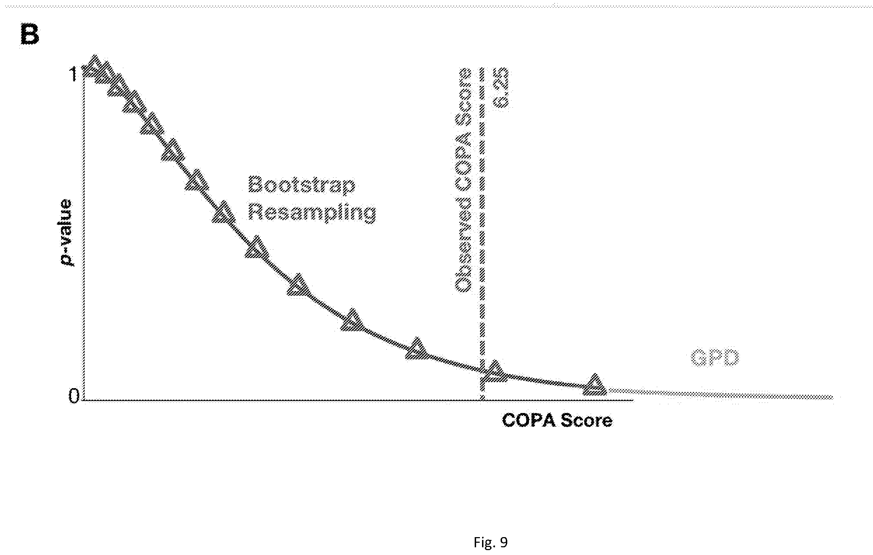

Fig. 9 is a schematic illustration that shows that the significance can of the observed COPA score can then be determined empirically for the Bootstrap Resampling method by determining the proportion of randomizations that yielded a COPA score as or more extreme. By fitting a Generalized Pareto Distribution (GPD), Bootstrap with GPD enables estimation of significance on the tail end of the spectrum with fewer randomizations through extrapolation of the p-value distribution.

Fig 13 and 14. Fitting of GPD to the tail distribution of resampled COPA scores. Fig. 13 shows tail of the empirical distribution (black line) and the fitted GPD (orange line). Fig. 14 shows good match between fitted p-values and resampled p-values before reaching the resolution limit.

Example 3

ssCOPA, a Binomial resampling method

While the p-value for a given observed COPA score can be obtained empirically through randomization testing, this becomes computationally intensive as assay set size increases. In addition, extremely significant p-values require an infeasible number of Bootstrap resampling tests to achieve the resolution necessary to estimate them, which again requires intensive computation. In order to address this, we introduce an exact solution that allows for the computation of p-value directly.

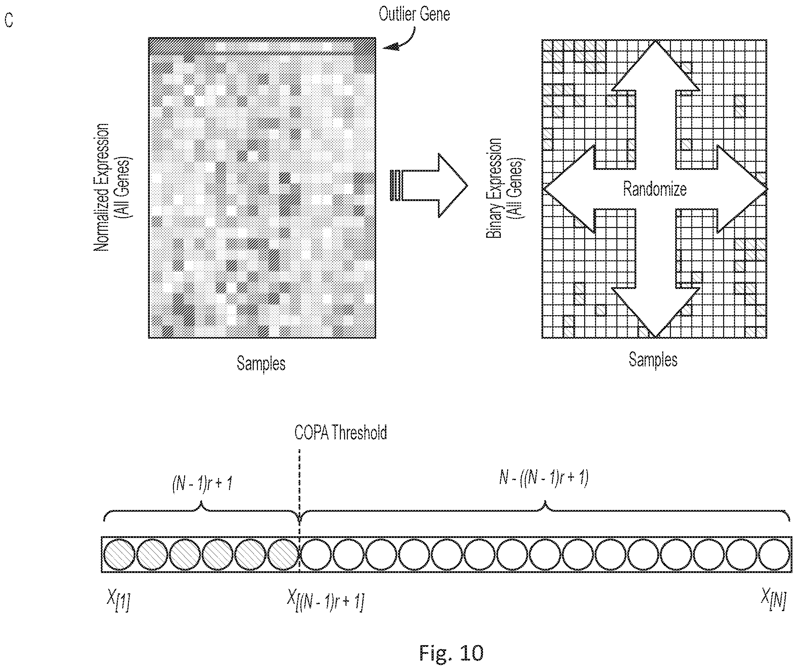

In randomization testing, the p-value for a given observed COPA score, k, is determined by the total number of bootstrapped COPA scores from equally or more significant than k divided by the total number of Bootstrap resampling tests. Bootstrapped COPA scores are calculated by randomizing the values across the dataset and obtaining the value at the qth percentile for each row. In order for a given bootstrapped COPA score to be equally or more significant than k, there must be at least a certain number of sampled values equally or more significant than k such that the qth percentile is also equally or more significant. The proportion of values equally or more significant than k required for a bootstrapped COPA score to be equally or more significant than k is $r = \min(q, 1-q)$, since q is required for lower-side and $1 - q$ is required upper-side. The number of values that corresponds to r depends on the calculation of percentile, in our implementation we use the R-7 estimation type, thus the value is the $((N-l)r+l)$th value in the sorted list of N values from most significant to least significant (Hyndman et al., 1996). This setup describes a Bernoulli trial with unequal probabilities since the probability of sampling a value equally or more significant than k is dependent on the column that value is being drawn from, Figure 10.

Fig. 10 is a schematic illustration of the binomial resampling method. As shown, binomial resampling provides an exact solution for computing the p-value for Bootstrap Resampling without the need for randomization by directly calculating the probability of randomly sampling a COPA score as or more extreme as the observed COPA score treating the process as a Bernoulli trial.

For a Bernoulli trial, we are able to calculate the probability of s number of successes occurring with probability p over N trials. A success, in this case, is defined as sampling a value equally or more significant than the observed COPA score k and the number of successes s needs to be at least $(N-1)r+1$. To match our Bootstrap resampling setup in which values are permuted across the dataset with replacement, the probability p of drawing a value equally or more significant than k is to the proportion of values in the data that satisfy this requirement. This setup can be described analytically by the Binomial distribution.

Comparison of accuracy of all three methods on two Breast cancer data sets

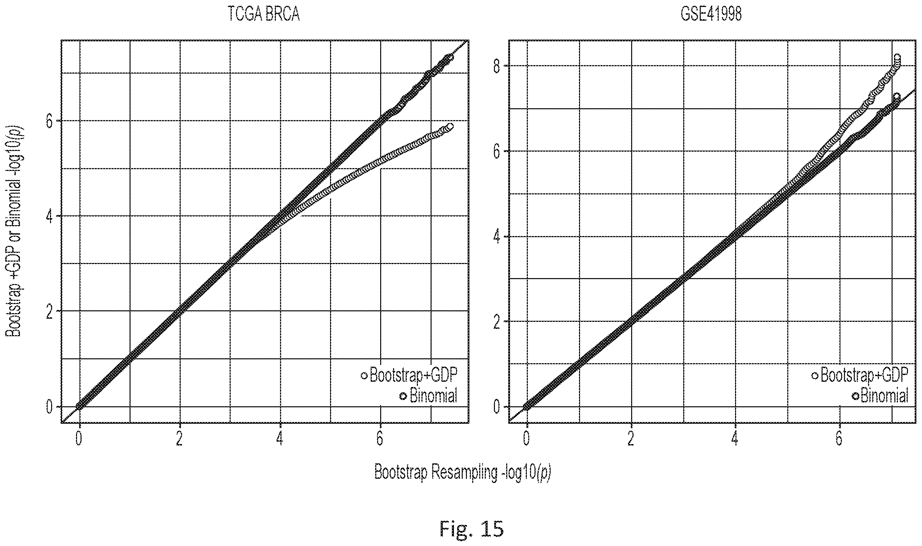

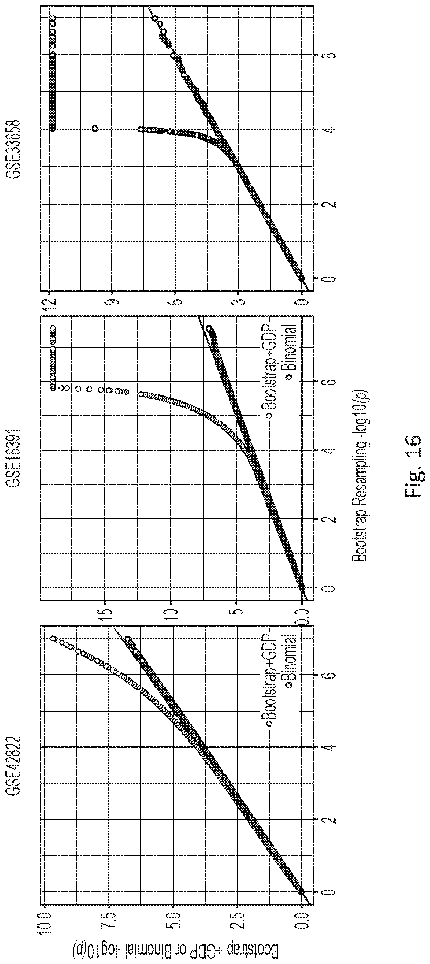

To evaluate the accuracy of each of the three methods, we used as a baseline Bootstrap resampling and compared it to the accuracy of Bootstrap + function and binomial resampling. Each method was applied to the five different breast cancer studies within the Illumina BaseSpaceTM Cohort Analyzer platform and compared against the empirically derived p-values from 10,000 Bootstrap resampling randomizations. We reduced randomizations to 100 for the Bootstrap + function method, which reduces computation time by 99%. The comparison of the log10(p-values) are plotted below in Fig 15 and in Fig. 16.

Fig. 11 shows performance of Binomial resampling method and Bootstrap + function compared to the Bootstrap resampled p-values. The orange line represents those from Binomial resampling method and the blue line represents the p-values from Bootstrap + function.

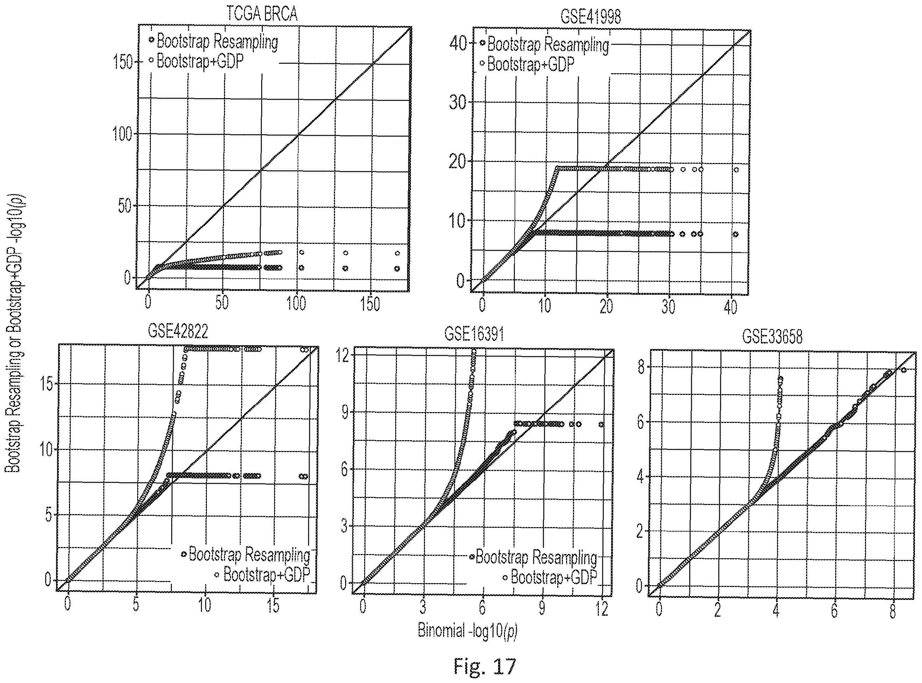

The Binomial method showed the best accuracy to the empirically derived p-values. In addition, the Bootstrap + function method is also becomes capped for more significant p-values, Figure 17. Thus, the Binomial method is the most suitable method significance assessment for improved accuracy and reduced computation time.

Accuracy of the Binomial method on TCGA Breast cancer data at low sample and feature size

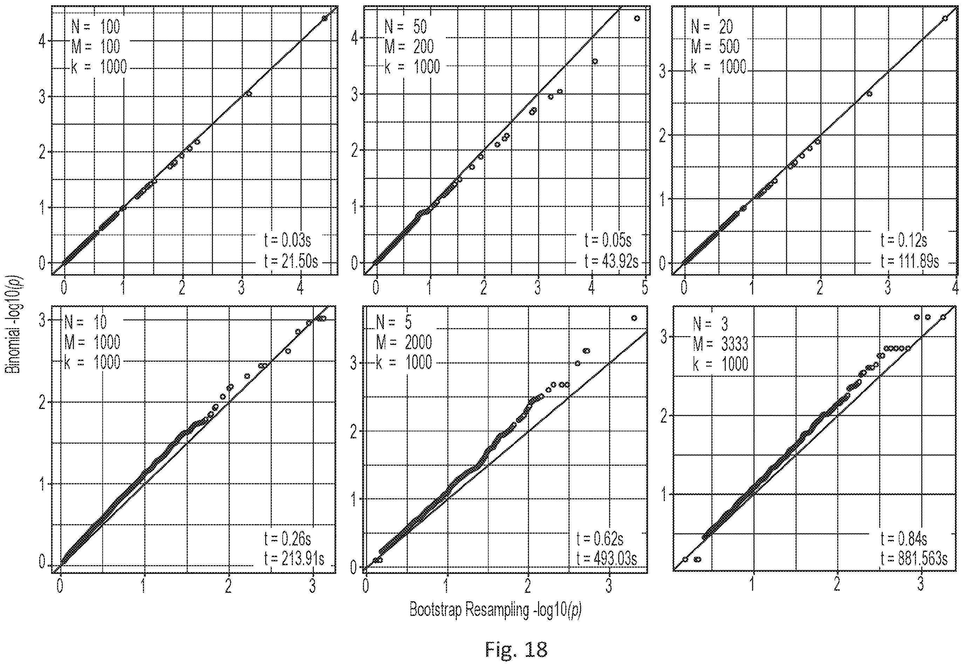

Due to differences in the approximation of COPA scores at low sample size, we suspect the p-values for the empirical and Binomial model may diverge. Figure 18 confirms this phenomenon, because at a low sample size greater variation in randomized COPA scores results as the likelihood of sampling an extreme value at any given percentile increases, especially as the COPA function relies more heavily on interpolation given a fewer number of points which differs from the approximation yielded from the continuous Beta function. Additionally, the compute time appears to grow linearly with the increasing number of statistical tests, one per feature, with fixed number of data points. The computation time is also significantly reduced for the Binomial derived p-values. Thus, compute time is referenced on Fig. 18 where an observation is that an increase in features (factors) leads to an increase in compute time for all methods, but the binomial method is orders of magnitude faster since it is independent of the number of randomizations. Said in another way the bootstrap resampling and the bootstrap function methods require more randomizations and more compute time to yield accurate numbers, whereas the binomial method is a direct computation that is not dependent on additional compute time to be accurate.

Meta-Analysis comparison of p-value based method against Binomial test

We downloaded five breast cancer studies from Illumina BaseSpaceTM Cohort Analyzer platform listed in Table 1, four studies are based on microarrays from Gene Expression Omnibus (GEO) and one is TCGA BRCA study using RNA-Seq technology. Unlike micro-array data, RNA-Seq produces zero values for many samples. The COPA algorithm is sensitive to the small standard deviation that arises in genes where more than 30% of samples have a zero value. Therefore, for each RNA-Seq data-set, we omit those genes that have zero expression value in more than 30% of samples. In addition to reduce spurious results, we omitted from micro-array and RNA-Seq the genes with bottom 20% mean expression.

We then applied the three methods for p-value calculation to these studies. After p-values for each study are generated, Fisher's combined p-value method, which is known to be statistically robust and sensitive in a meta-analysis method comparison [9], is used to perform the meta-analysis. We compared this to four types of meta-analyses performed on COPA scores. First, we applied a binomial test, which is commonly used in meta-COPA analysis [8]. We also evaluated three existing rank-based methods, including median rank, mean rank and product rank [Chang].

To evaluate the performance of different p-value calculation methods, we used the list of top 23 genes discovered by ONCOMINE via a large-scale meta-COPA analysis across 31 breast cancer studies [8] as a validation gene set. We adopted the Running Fisher (RF) test (Kupershmidt et al. 2010) to assess how consistent each testing gene list generated by one of the meta-analysis methods matches the ONCOMINE gene list. The Running Fisher method scan the testing gene list and when reaching each matched gene from the validation set, Fisher's exact test is performed to generate p-values. Finally, the minimum p-value is obtained to measure the concordance of the testing gene list with the validation set. This method evaluates the gene enrichment between two sets, similar to Gene Set Enrichment Analysis (GSEA), but is more flexible and sensitive than GSEA.

We first evaluated the consistency of gene lists generated from individual studies with the ONCOMINE gene list (see Table 1). Clearly, the larger the sample size of the study, the smaller the Running Fisher p-value. Next, we performed meta-analysis of the five studies using various methods (see Table 2). Binomial test mainly uses the absence / presence information of genes among top 1% list across independent studies. It usually requires a large number of studies to achieve sufficient sensitivity. As expected, with five studies Binomial test performs the worst with RF p-value at 2.95e-14 as many genes have the identical p-values from Binomial test. Fisher's combined p-values using various p-value generating methods all perform reasonably well, among which Bootstrap + function outperforms all the methods. Fisher's combined p-values with Binomial method achieve both high accuracy and computational efficiency. Rank-based methods also perform well, with product rank outperforming median rank or mean rank. Last, we also evaluated the stability and robustness of the meta-analysis by removing every one of the five studies to see if there is one study that dominates the results (see Table 3). These p-values are generally stable for these methods after removing each of the five studies.

| Individual study (COPA 95%) | Sample Size | Running Fisher *p*-value |
|---|---|---|
| TCGA BRCA | 1044 | 3.84E-22 |
| GSE41998 | 279 | 3.77E-19 |
| GSE42822 | 91 | 4.98E-14 |
| GSE16391 | 55 | 2.70E-15 |
| GSE33658 | 11 | 2.81E-05 |

Table 1. List of five breast cancer studies. The first column shows the names of the five breast cancer studies. The second column shows the sample size of each study. The third column shows the running Fisher p-value of each study to evaluate consistency with ONCOMINE gene list.

| Meta-analysis methods (COPA 95%) of five breast cancer studies | Running Fisher $p$-value |
|---|---|
| Binomial test | 2.954229e-14 |
| Fisher's combined $p$-values with Bootstrap | 4.746938e-21 |
| Fisher's combined $p$-values with Binomial method | 1.306501e-17 |
| Fisher's combined $p$-values with Bootstrap + GPD | 3.025889e-24 |
| Median Rank | 2.131294e-23 |
| Mean Rank | 7.220085e-19 |
| Product Rank | 9.791574e-24 |

Table 2. Performance of various methods used for meta-analysis of five breast cancer studies. The first column shows the method used for meta-analysis of the five breast cancer studies. The second column shows the RF p-value of meta-analysis using each method to evaluate consistency with ONCOMINE gene list. The third column shows the computation time for each method.

| Meta-analysis methods of four breast cancer studies (COPA 95%) | Remove GSE33658 (11) | Remove GSE16391 (55) | Remove GSE42822 (91) | Remove GSE41998 (279) | Remove TCGA (1044) |
|---|---|---|---|---|---|
| Fisher's combined $p$-values with Binomial method | 1.31E-17 | 2.42E-16 | 3.99E-17 | 3.49E-17 | 2.48E-19 |
| Fisher's combined $p$-values by Bootstrap | 2.54E-24 | 2.48E-19 | 2.32E-17 | 7.17E-21 | 1.78E-14 |
| Median Rank | 3.97E-22 | 9.69E-16 | 3.05E-17 | 1.57E-23 | 2.56E-15 |
| Mean Rank | 1.37E-19 | 6.07E-18 | 1.94E-16 | 6.22E-20 | 2.19E-14 |
| Product Rank | 6.28E-28 | 7.32E-20 | 2.13E-23 | 1.22E-24 | 7.45E-15 |

Table 3. Stability evaluation of various meta-analysis methods. The first column shows the method used for meta-analysis of four breast cancer studies. The rest five columns show the RF p-value after removing each of the five studies.

Discussion

Since its introduction, Meta-COPA analysis has been a popular approach in identifying molecular markers for targeted diseases. Current solutions to performing meta-analyses have the shortcoming that they require a large number of studies. In addition, within a single study no significance is currently being addressed to each COPA score, making it impossible to know which outlier genes are statistically significant. We solve these problems by introducing three methods that assess the significance of individual COPA scores, including Bootstrap resampling, Bootstrap + function, and Binomial method. Bootstrap resampling is a standard approach to generating p-values but is computational intensive. The Bootstrap + function method, which works by approximating the tail distribution of COPA scores based on a much smaller number of randomizations of studies, estimates p-values conservatively, particularly for top genes. The analytical solution, Binomial resampling method, allows estimation of the p-value without the need for randomization tests, thus is computational succinct. Furthermore, the Binomial method yields the most accurate results showing minimal deviations from the empirical p-values. Binomial method is recommended to be implemented in Illumina BaseSpaceTM Cohort Analyzer platform for accuracy and performance.

In practice, these significance assessment methods we developed still have some areas to improve, including relaxing the assumptions for those tests. For example, COPA method scans one percentile each time to detect genes with outlier patterns. However, when identifying genes with outlier subjects, fixing one percentile for COPA scores may undermine the sensitivity of COPA method. For example, in prostate cancer subjects, ERG gene is significantly upregulated in 35% of subjects (See S6 Fig.). If we only scan the 90th or 95th percentile, ERG is buried under thousands of genes. As our significance assessment approach enables fair comparison between different parameters, e.g., percentiles, investigators can now scan multiple percentiles (e.g. 60%,65%,...,90%,95% for upper tails and 5%,10%,..., 40% for lower tails) and select the percentile with smallest p-value to represent a specific gene. This will boost the sensitivity of detecting heterogeneous subsets of gene expression of molecular markers.

Another potential extension of current method is to adjust for outlier patterns in normal samples. Current p-value calculation is pooling all the genes assuming these genes behave similarly and independently in subjects. In reality, genes might have distinct patterns even in normal samples. Some genes might have remarkable variation in normal, causing spurious outlier signals in subjects. Adjusting for outlier patterns in normal is quite critical in generating precise p-values for COPA scores, particularly for highly variable genes.

In theory, our methods can not only be applied to RNA expression, but to any continuous measures with proper normalization and standardization, including protein expression, miRNA expression, and other clinical measurements such as cholesterol values. The generated p-values are flexible and can be compared across studies or parameters. This then allows us to perform meta-analysis using Fisher's combined p-value method. We anticipate that our methods will improve the identification of molecular markers to facilitate personalized medicine.

Materials and Methods

Expression data and preprocessing

Expression datasets were obtained through the Illumina BaseSpaceTM Cohort Analyzer platform which includes data curated from Gene Expression Omnibus (GEO) and published cohorts from The Cancer Genome Atlas (TCGA). Datasets utilized for this analysis include four breast cancer microarray studies from GEO, one breast cancer RNA-Seq cohort, and one TCGA prostate cancer RNA-Seq cohort from TCGA. Prior to COPA analysis, the data is preprocessed by filtering out genes with low expression values likely to resulting in false positives and values are log transformed to reduce the skewness resulting in more comparable magnitudes for COPA scores in the lower and upper percentiles. Firstly, genes among the bottom 20% of low expressors by mean are removed for all data types. Secondly, genes with >30% of samples with an RPKM value of zero are removed for RNA-Seq data. Lastly, the data is log2 transformed after adding 0.01 to each value to result in a more symmetric distribution of values for expression data types. The addition of 0.01 is to prevent collisions involving the log of zero.

Percentile estimation function

In most situations, the percentile does not land on a discrete index for the COPA calculation. Interpolating the percentile value is standard practice in these cases, however, there are several interpolation strategies employed under different situations. For our COPA implementation, we utilize the R-7 estimation type over other estimation types because it results in a smooth function for $r \in [0, 1]$ over indices from 1:N. Other estimation types, such as R-6, become flat at the tails resulting in reduced resolution between percentile values at the extremes.

For the R-7 estimation type, the percentile is calculated by identifying the hth value in the sorted array of values. The index h is computed as:

$$h = (N - 1)r + 1$$

When the index h does not fall on an integer value the interpolation of the percentile value is handled as:

$$Q_r = x_{\lfloor h \rfloor} + (h - \lfloor h \rfloor)\left(x_{\lfloor h \rfloor + 1} - x_{\lfloor h \rfloor}\right)$$

Bootstrap resampling

In order to evaluate the significance of COPA values within a single study, we test the null hypothesis that no outlier samples exist in our cohort populations, therefore all genes should be independent and identically distributed across all samples. In order to test this hypothesis, we perform randomization to compute the statistical deviation of our observed data against the null model, however, randomization within each row is insufficient for COPA since the measurement of COPA score is performed row-wise. To perform dataset-wise randomization, we make the assumption that each value for a given measurement platform follows the same overall distribution. For most measurement platforms, such as RNA-Seq or microarray, values between genes are not directly comparable due to differences in base levels of expression or chemistry. To solve this problem the standardization utilized by COPA is applied to scale the values according to median and median absolute deviation prior to randomization. Resampling of the standardized values randomly with replacement allows us to generate the null distribution for COPA values. Given a sufficiently large dataset, we make the assumption that the standardized values in the dataset are representative of the entire population in which these samples were derived.

Tail approximation of bootstrap resampling by function of generalized Pareto distribution (GPD)

To achieve high resolution of p-values for extreme COPA values, an intractable number of randomizations would be necessary. Due to this high computational cost, we explored tail approximation using a function such as, for example, generalized Pareto distribution (GPD). The approximated p-value of this 0.1% upper tail is calculated as $f_t$ * $(1 - F_{GPD}(x - t))$, where $f_t$ represents the fraction of scores in the tail, i.e., the fraction of scores $\geq t$ ($t$ is the threshold value of the tail), and in this example $f_t$ = 0.1%. $F_{GPD}$ represents the cumulative distribution of GPD.

Binomial resampling method

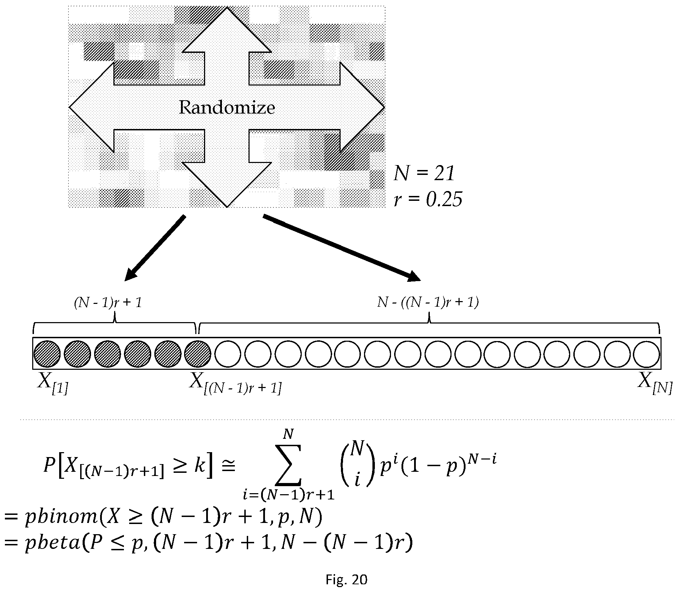

The unique combination of randomization and percentile calculation following standardization in our Bootstrap Resampling model can be computed analytically without the need for repeated randomizations. The exact solution using the Binomial cumulative distribution for p-value estimation follows (see also Fig. 20):

$$P\big[X_{[(N-1)r+1]} \geq k\big] \cong \sum_{s=(N-1)r+1}^{N} \binom{N}{s} p^s (1-p)^{N-s}$$

$$= pbinom(X \geq (N-1)r+1, p, N)$$

$$= pbeta(P \leq p, (N-1)r+1, N-(N-1)r)$$

Since the Binomial cumulative distribution is related to the regularized incomplete beta distribution in the equation above, it is used for calculation of the Binomial distribution, non-integer values of (N-1)r+1 can be estimated directly without the need for a weighted geometric mean (Press et al., 1992).

In addition or as an alternative to the above disclosure on binomial resampling, the binomial cumulative distribution for p-value estimation can be as follows (see Fig. 21):

$$P\big[X_{[(N-1)r+1]} \geq k\big] \cong \sum_{i=(N-1)r+1}^{N} \binom{N}{i} p^i (1-p)^{N-i}$$
$$= pbinom(X \geq (N-1)r+1, p, N)$$
$$= pbeta(P \leq p, (N-1)r+1, N-(N-1)r)$$

Fig. 8 depicts that the Bootstrap Resampling and Bootstrap with function methods approximate the significance of a COPA score through randomizations. Random draws can be obtained through the bootstrapping, random sampling with replacement, of the normalized expression matrix then calculating COPA scores for those randomizations. The red bar indicates sampled values that are as or more extreme than the observed COPA score. Note that a randomly sampled COPA score is more extreme than the observed score only when the number of sampled extreme values (red bar) exceeds the COPA threshold (dashed line), this is a key observation that will enable an exact calculation via binomial resampling.

Fig. 9 shows that the significance of the observed COPA score can be determined empirically for the Bootstrap Resampling method by determining the proportion of randomizations that yielded a COPA score as or more extreme.

By fitting a Generalized Pareto Distribution (GPD), Bootstrap with GPD enables estimation of significance on the tail end of the spectrum with fewer randomizations through extrapolation of the p-value distribution.

Fig. 10 shows that binomial resampling provides an exact solution for computing the p-value for Bootstrap Resampling without the need for randomization by directly calculating the probability of randomly sampling a COPA score as or more extreme as the observed COPA score treating the process as a Bernoulli trial.

Fig. 11 shows a random shuffling compared with a bootstrap resampling as it relates to TCGA BRCA gene.

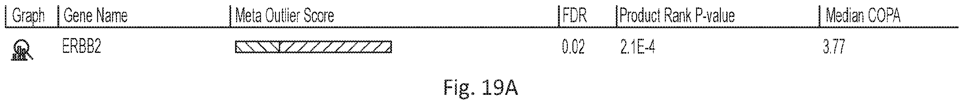

Fig. 19A shows that ERBB2 with Q-value = 0.02 is among multiple testing significant results (but would not be in a single study).

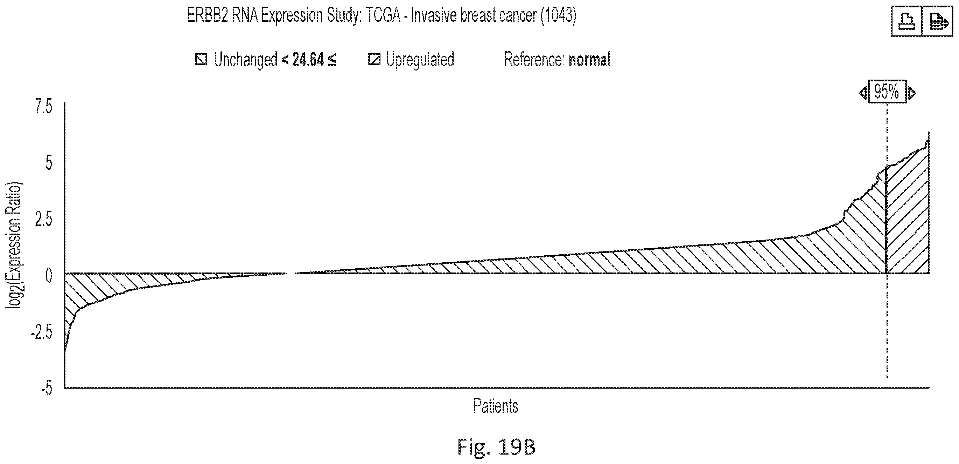

Fig. 19B shows a waterfall plot of ERBB2 expression in one of the five studies (TCGA).

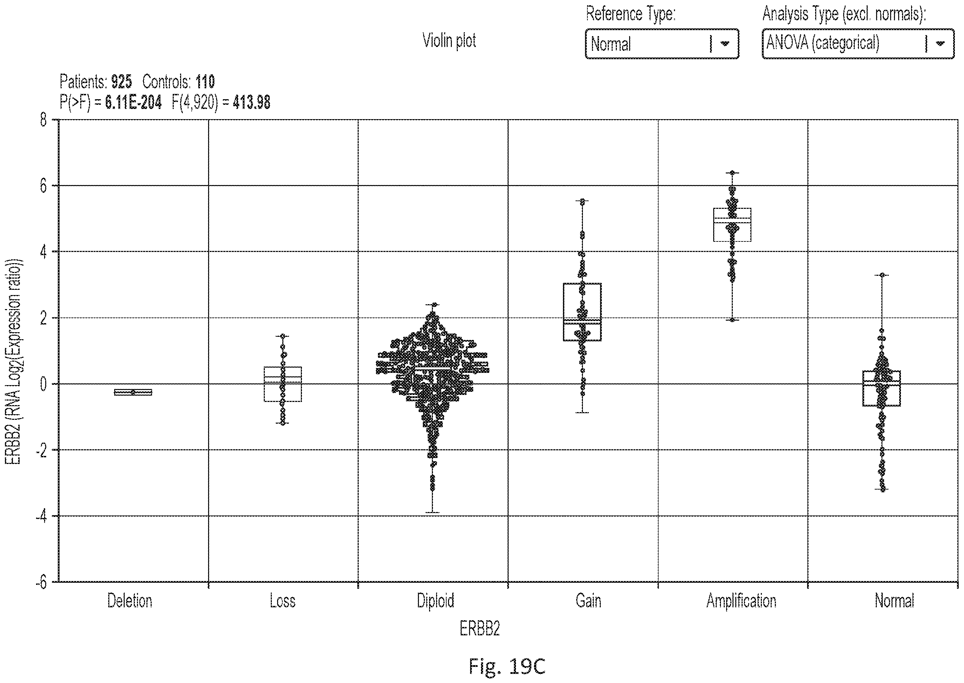

Fig. 19C shows ERBB2 expression (2log of fold change, y-axis) plotted against Copy Number Variation (x-axis) shows ERBB2 outlier overexpression is likely explained by Amplification, as the violin plots show that ERBB2 subjects with >3 copies explain most of the high expressors.

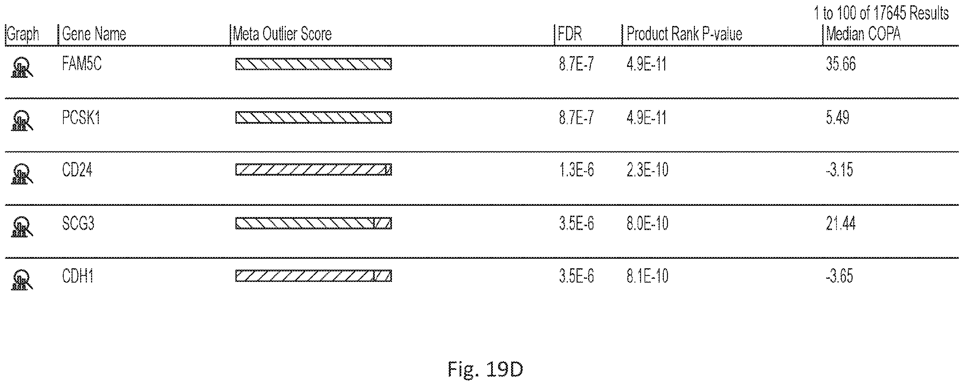

Fig. 19D shows meta-analysis of five BRC studies identifies FAM5C as most significant Outlier gene (using product rank).

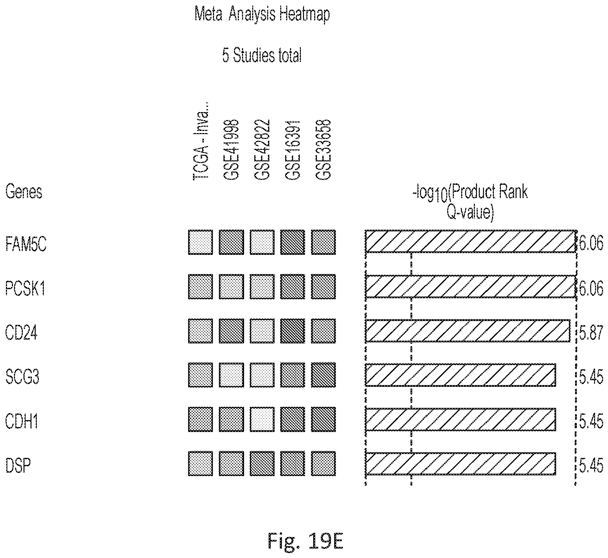

Fig. 19E shows a heatmap showing the normalized rank score for each gene across all studies of the meta analysis, with log10 of the Q-value.

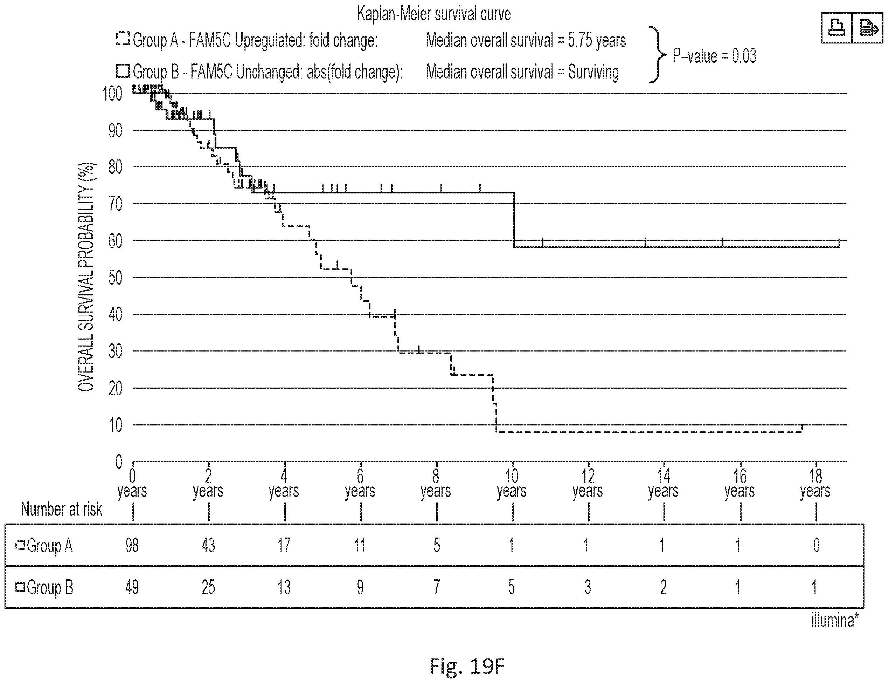

Fig. 19F shows survival analysis in TCGA shows that outlier subjects with >7 fold upregulation of FAM5C vs those with no major fold change (<2), have poorer Overall Survival.

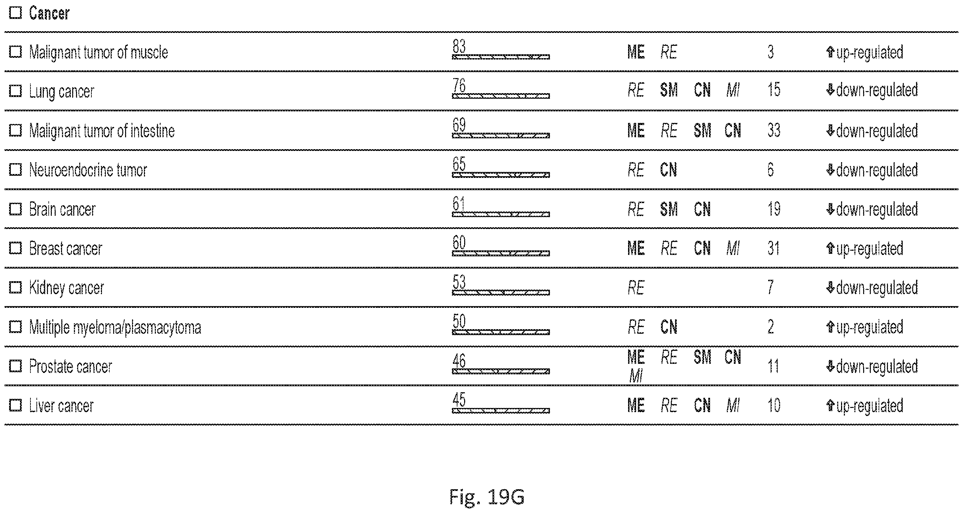

Fig. 19G shows BaseSpace Correlation Engine Disease Atlas app showing that there are 31 public Breast cancer studies in which FAM5C is also up-regulated.

Thus, a detection system for identifying genes with outlier expression across multiple samples, can include at least one processor; and at least one non-transitory computer readable medium containing instructions that, when executed by the at least one processor, cause the at least one processor to perform operations. These operations can include: receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data; determining a null distribution of the distribution statistic using the standardized gene expression data; and outputting a significance value of the genes across the multiple samples, the significance value based on the value of the distribution statistic and the null distribution.

The detection system can detect according to the principles of the bootstrap resampling method, wherein determining the value of the distribution statistic includes bootstrap resampling comprising performing randomized iterations of shuffling the gene expression data that generates newly assigned gene expression values for each gene. With this approach, the probability of outlier gene expression data can be calculated from the observed and randomized gene expression values.

The detection system can also be configured such that the randomly selected observations are randomly selected from the standardized observational data with replacement. The detection system can be configured such that the bootstrap resampling includes randomized iterations for all possible combinations of randomized gene expression values.

The distribution statistic can include a quantile, such as 75%.

The detection system can work in accordance with the bootstrap resampling with function approach. The step of determining the value of the distribution statistic can include: generating bootstrapped values for a gene by randomizing a portion of all possible randomized iterations for the standardized gene expression data, and fitting a function to at least a portion of the bootstrapped values to estimate a tail of the null distribution for the gene, the tail including outlier data for the significance value. The probability of outlier gene expression data can be calculated from the estimated tail. The function can be a continuous probability distribution parameterized by at least one of a scale parameter and a shape parameter. The function can be a generalized Pareto distribution.

In accordance with embodiments of the invention, operations can further include receiving additional significance values for the gene and outputting a revised significance value for the gene based on the received additional significance values for the gene and the significance value for the gene.

The system can be implemented according to distributed architecture comprising a controller that assigns tasks to workers.

The detection system can be implemented using the principles of the binomial method. In this embodiment, determining the value of the distribution statistic can include for each sample, calculating a probability of a gene being expressed at or above a predetermined threshold using Bernoulli trials based on the total number of samples and a percentile cutoff for the gene.

The calculating the probability of the sample can include binarizing the values in accordance with the formula

$$P\big[X_{[(N-1)r+1]} \geq k\big] \cong \sum_{i=(N-1)r+1}^{N} \binom{N}{i} p^{i}(1-p)^{N-i}$$
$$= pbinom(X \geq (N-1)r+1, p, N)$$
$$= pbeta(P \leq p, (N-1)r+1, N-(N-1)r)$$

,

where k = outlier COPA score, N = number of samples, r = outlier quantile, and p = probability of a draw $\geq$ k.

The null significance likelihood can further depend on whether the quantile is an upper quantile or lower quantile. The distribution can include a cumulative binomial distribution.

The operations can include receiving additional significance values for the factor and outputting a revised significance value for the factor based on the received additional significance values for the factor and the significance value for the factor.

The system can be implemented using a parallel computing architecture. The distribution can include an incomplete beta distribution.

A non-transitory computer readable medium for identifying genes with outlier expression across multiple samples, can include instructions that, when executed by the at least one processor, cause the at least one processor to perform operations. The operations can include: receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data; determining a null distribution of the distribution statistic using the standardized gene expression data; and outputting a significance value of the genes across the multiple samples, the significance value based on the value of the distribution statistic and the null distribution.

A computer-implemented method for identifying genes with outlier expression across multiple samples, can include: receiving gene expression data of a plurality of samples, the samples comprising gene expression values corresponding to genes; standardizing the gene expression data using the median and median absolute deviation of each gene; determining a value of a distribution statistic for the standardized gene expression observations based on a probability of outlier gene expression data; determining a null distribution of the distribution statistic using the standardized gene expression data; and outputting a significance value of the genes across the multiple samples, the significance value based on the value of the distribution statistic and the null distribution.

Figs. 7A depicts a schematic of an exemplary computing device 700 for implementing the disclosed embodiments. The components of system 100, such as user device 120, analysis system 110, and data system 105, may generally be implemented as one or more of computing device 700. According to some embodiments, the exemplary computing device 700 may include a processor 705, memory 710, display 715, I/O interface(s) 720, and network adapter 725. These units may communicate with each other via bus 730, or wirelessly. The components shown in Fig. 7 may reside in a single device or multiple devices.

Processor 705 may be one or more microprocessors, central processing units, or graphics processing units performing various methods in accordance with disclosed embodiments. These processing units may include one or more cores. Memory 710 may include one or more computer hard disks, random access memory, removable storage, or remote computer storage. In various embodiments, memory 710 stores various software programs executed by processor 705. Display 715 may be any device that provides a visual output, for example, a computer monitor, an LCD screen, etc. I/O interfaces 720 may include keyboard, a mouse, an audio input device, a touch screen, or similar human interface device. Network adapter 725 may include hardware and/or a combination of hardware and software for enabling computing device 700 to exchange information with external networks. As a non-limiting example, network adapter 725 may include a wireless wide area network (WWAN) adapter, a Bluetooth module, a near field communication module, or a local area network (LAN) adapter.

Fig. 7B depicts a schematic of an exemplary system for distributed computation of significance values, consistent with disclosed embodiments. In such embodiments, system 100 may be implemented using a parallel computing architecture. This parallel computing architecture may be implemented using one or more computing devices as described according to Fig. 7A. In such an architecture (e.g., Hadoop or the like), one or more components of system 100 may comprise controller 740 and workers 745. Caller 735 may comprise a component of system 100 that requests a computational result using this parallel computing architecture. In some embodiments, caller 735 may be configured to communicate with controller 740, or another element of the parallel computing architecture, to request the computational result. Controller 740 may comprise one or more nodes of the parallel computing cluster configured with data and instructions for managing performance of the distributed computation. Controller 740 may be configured to assign nodes of the parallel computing cluster as workers 745. Workers 745 may include one or more nodes of the parallel computing cluster configured with data and instructions for performing distributed computations. Workers 745 may include mappers and reducers. The mappers may be configured to group data elements, such as observational values, for aggregation by the reducers. The reducers may be configured to compute aggregate results for each group of mapped data elements. Controller 740 may be configured to assign worker nodes as mappers and reducers. Controller 740 may also be configured to track the status of workers 745, assigning and reassigning tasks (such as mapping and reduction) to worker nodes as needed. Controller 740 may further be configured to provide the result of the distributed computation to caller 735. As would be recognized by one of skill in the art, other parallel computing implementations are possible and the above disclosure is not intended to be limiting.

The foregoing disclosed embodiments have been presented for purposes of illustration only. This disclosure is not exhaustive and does not limit the claimed subject matter to the precise embodiments disclosed. Those skilled in the art will appreciate from the foregoing description that modifications and variations are possible in light of the above teachings or may be acquired from practicing the inventions.

References

1. Tomlins SA, Rhodes DR, Perner S, Dhanasekaran SM, Mehra R, et al. Recurrent fusion of TMPRSS2 and ETS transcription factor genes in prostate cancer. Science 2005 310, 644-648.
2. Rhodes DR, Yu J, Shanker K, Deshpande N, Varambally R, et al. ONCOMINE: A Cancer Microarray Database and Integrated Data-Mining Platform. Neoplasia 2004 6(1): 1-6.
3. Tibshirani R, Hastie T. Outlier sums for differential gene expression analysis. Biostatistics 2006 8, 2-8.
4. Wu B. 2007. Cancer outlier differential gene expression detection. Biostatistics 2007 8(3): 566-575.
5. Lian H. MOST: detecting cancer differential gene expression. Biostatistics 2008 9(3): 411-418.
6. de Ronde JJ, Rigaill G, Rottenberg S, Rodenhuis S, Wessels LF. Identifying subgroup markers in heterogeneous populations. Nucleic Acids Research 2013 41(21) e200
7. Wang C, Taciroglu A, Maetschke SR, Nelson CC, Ragan MA, et al. mCOPA: analysis of heterogeneous features in cancer expression data. J Clin Bioinforma. 2012 2: 22.
8. Rhodes DR, Ateeq B, Cao Q, Tomlins SA, Mehra R, et al. AGTR1 overexpression defines a subset of breast cancer and confers sensitivity to losartan, an AGTR1 antagonist. Proc. Natl. Acad. Sci. 2009 106(25):10284-10289.
9. Chang LC, Lin, HM, Sibille E, Tseng GC. Meta-analysis methods for combining multiple expression profiles: comparisons, statistical characterization and an application guideline. BMC Bioinformatics 2013 14:368.
10. Edgington E. Randomization Tests. Marcel Dekker, Inc. 1980.
11. Knijnenburg TA, Wessels LFA, Reinders MJT, Shmulevich I. Fewer permutations, more accurate P-values. Bioinformatics 2009 25: 161-168.
12. Gumbel EJ. Statistics of extremes. Columbia University Press, New York 1958.
13. Kupershmidt I, Su QJ, Grewal A, Sundaresh S, Halperin I, et al. Ontology-Based Meta-Analysis of Global Collections of High-Throughput Public Data. PLoS ONE 2010 5(9): e13066.
14. Heskes T, Eisinga R, Breitling R. A fast algorithm for determining bounds and accurate approximate p-values of the rank product statistic for replicate experiments. BMC Bioinformatics 2014 15(367).
15. Hyndman RJ and Fan Y. Sample quantiles in statistical packages. The American Statistician 1996, 50(4): 361-365.
16. Press WH, Flannery BP, Teukolsky SA, and Vetterling WT. Numerical Recipes in C: The Art of Scientific Computing. Press Syndicate of the University of Cambridge 1992, Second Edition.

## Claims

1. A detection system for identifying genes with outlier expression across multiple samples, comprising:

   at least one processor; and
   at least one non-transitory computer readable medium containing instructions that, when executed by the at least one processor, cause the at least one processor to perform operations comprising:

   receiving gene expression data of a plurality of samples, the samples comprising gene expression values corresponding to genes;
   standardizing the gene expression data using the median and median absolute deviation of each gene;
   determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene; determining a null distribution of the distribution statistic using the standardized gene expression data; and outputting a significance value of the genes across the multiple samples, the significance value based on the value of the distribution statistic and the null distribution.

2. The detection system of claim 1, wherein the distribution statistic comprises a quantile.

3. The detection system of claim 1, wherein the operations further include receiving additional significance values for the gene and outputting a revised significance value for the gene based on the received additional significance values for the gene and the significance value for the gene.

4. The detection system of claim 1, wherein the system is implemented according to distributed architecture comprising a controller that assigns tasks to workers.

5. The detection system of claim 1, wherein calculating the probability of the sample includes binarizing the values in accordance with the formula

$$P\big[X_{[(N-1)r+1]} \geq k\big] \cong \sum_{s=(N-1)r+1}^{N} \binom{N}{s} p^s (1-p)^{N-s}$$

$$= pbinom(X \geq (N-1)r+1, p, N)$$

$$= pbeta(P \leq p, (N-1)r+1, N-(N-1)r)$$

where: $k$ = outlier COPA score, $N$ = number of samples, $r$ = outlier quantile, and $p$ = probability of a draw > k.

6. The detection system of claim 1, wherein the null significance likelihood further depends on whether the quantile is an upper quantile or lower quantile.

7. The detection system of claim 1, wherein the distribution comprises a cumulative binomial distribution.

8. The detection system of claim 1, wherein the operations further include receiving additional significance values for the factor and outputting a revised significance value for the factor based on the received additional significance values for the factor and the significance value for the factor.

9. The detection system of claim 1, wherein the system is implemented using a parallel computing architecture.

10. The detection system of claim 1, wherein the distribution comprises an incomplete beta distribution.

11. A non-transitory computer readable medium for identifying genes with outlier expression across multiple samples, comprising instructions that, when executed by the at least one processor, cause the at least one processor to perform operations comprising:

receiving gene expression data of a plurality of samples, the samples comprising gene expression values corresponding to genes;
standardizing the gene expression data using the median and median absolute deviation of each gene;
determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene; determining a null distribution of the distribution statistic using the standardized gene expression data; and outputting a significance value of the genes across the multiple samples, the significance value based on the value of the distribution statistic and the null distribution.

12. A computer-implemented method for identifying genes with outlier expression across multiple samples, comprising:

receiving gene expression data of a plurality of samples, the samples comprising gene expression values corresponding to genes;

standardizing the gene expression data using the median and median absolute deviation of each gene;

determining a value of a distribution statistic for the standardized gene expression observations based on a probability of outlier gene expression data, wherein the determining the value of the distribution statistic comprises: for each sample, calculating a probability of a gene being expressed at or above a predetermined threshold using Bernoulli trials based on the total number of samples and a percentile cutoff for the gene;

determining a null distribution of the distribution statistic using the standardized gene expression data; and

outputting a significance value of the genes across the multiple samples, the significance value based on the value of the distribution statistic and the null distribution.

**Patentansprüche**

1. Detektionssystem zum Identifizieren von Genen mit Ausreißer-Expression über mehrere Proben, das Folgendes umfasst:

   zumindest einen Prozessor; und
   zumindest ein nichtflüchtiges computerlesbares Medium, das Befehle enthält, die, wenn sie durch den zumindest einen Prozessor ausgeführt werden, bewirken, dass der zumindest eine Prozessor Operationen durchführt, die Folgendes umfassen:

   Empfangen von Genexpressionsdaten einer Vielzahl von Proben, wobei die Proben Genexpressionswerte umfassen, die Genen entsprechen;
   Standardisieren der Genexpressionsdaten unter Verwendung des Medians und der mittleren absoluten Abweichung vom Median jedes Gens;
   Bestimmen eines Werts einer Verteilungsstatistik für die standardisierten Genexpressionsbeobachtungen basierend auf einer Wahrscheinlichkeit von Ausreißer-Genexpressionsdaten, wobei das Bestimmen des Werts der Verteilungsstatistik Folgendes umfasst: für jede Probe Berechnen einer Wahrscheinlichkeit, dass ein Gen an oder über einem vorbestimmten Schwellenwert exprimiert wird, unter Verwendung von Bernoulli-Versuchen basierend auf der Gesamtanzahl von Proben und eines perzentilen Grenzwerts für das Gen;
   Bestimmen einer Nullverteilung der Verteilungsstatistik unter Verwendung der standardisierten Genexpressionsdaten; und
   Ausgeben eines Signifikanzwerts der Gene über die mehreren Proben, wobei der Signifikanzwert auf dem Wert der Verteilungsstatistik und der Nullverteilung basiert.

2. Detektionssystem nach Anspruch 1, wobei die Verteilungsstatistik ein Quantil umfasst.

3. Detektionssystem nach Anspruch 1, wobei die Operationen weiters das Empfangen zusätzlicher Signifikanzwerte für das Gen und das Ausgeben eines revidierten Signifikanzwerts für das Gen basierend auf den empfangenen zusätzlichen Signifikanzwerten für das Gen und dem Signifikanzwert für das Gen umfassen.

4. Detektionssystem nach Anspruch 1, wobei das System gemäß einer verteilten Architektur, umfassend eine Steuerung, die Worker-Modulen Aufgaben zuweist, implementiert ist.

5. Detektionssystem nach Anspruch 1, wobei das Berechnen der Wahrscheinlichkeit der Probe das Binarisieren der Werte gemäß folgender Formel umfasst:

$$P\big[X_{[(N-1)r+1]} \geq k\big] \cong \sum_{s=(N-1)r+1}^{N} \binom{N}{s} p^s (1-p)^{N-s}$$

$$= pbinom(X \geq (N-1)r + 1, p, N)$$

$$= pbeta(P \leq p, (N-1)r + 1, N - (N-1)r)$$

worin: $k$ = Ausreißer-COPA-Score, $N$ = Anzahl von Proben, $r$ = Ausreißer-Quantil und $p$ = Ziehungswahrscheinlichkeit > k ist.

6. Detektionssystem nach Anspruch 1, wobei die Nullsignifikanz-Wahrscheinlichkeit weiters davon abhängt, ob das Quantil ein oberes Quantil oder ein unteres Quantil ist.

7. Detektionssystem nach Anspruch 1, wobei die Verteilung eine kumulative Binomialverteilung umfasst.

8. Detektionssystem nach Anspruch 1, wobei die Operationen weiters das Empfangen zusätzlicher Signifikanzwerte für den Faktor und das Ausgeben eines revidierten Signifikanzwerts für den Faktor basierend auf den empfangenen zusätzlichen Signifikanzwerten für den Faktor und dem Signifikanzwert für den Faktor umfassen.

9. Detektionssystem nach Anspruch 1, wobei das System unter Verwendung einer parallelen Rechnerarchitektur implementiert ist.

10. Detektionssystem nach Anspruch 1, wobei die Verteilung eine inkomplette Betaverteilung umfasst.

11. Nichtflüchtiges computerlesbares Medium zum Identifizieren von Genen mit Ausreißer-Expression über mehrere Proben, umfassend Befehle, die, wenn sie durch den zumindest einen Prozessor ausgeführt werden, bewirken, dass der zumindest eine Prozessor Operationen durchführt, die Folgendes umfassen:

Empfangen von Genexpressionsdaten einer Vielzahl von Proben, wobei die Proben Genexpressionswerte umfassen, die Genen entsprechen;
Standardisieren der Genexpressionsdaten unter Verwendung des Medians und der mittleren absoluten Abweichung vom Median jedes Gens;
Bestimmen eines Werts einer Verteilungsstatistik für die standardisierten Genexpressionsbeobachtungen basierend auf einer Wahrscheinlichkeit von Ausreißer-Genexpressionsdaten, wobei das Bestimmen des Werts der Verteilungsstatistik Folgendes umfasst: für jede Probe Berechnen einer Wahrscheinlichkeit, dass ein Gen an oder über einem vorbestimmten Schwellenwert exprimiert wird, unter Verwendung von Bernoulli-Versuchen basierend auf der Gesamtanzahl von Proben und eines perzentilen Grenzwerts für das Gen;
Bestimmen einer Nullverteilung der Verteilungsstatistik unter Verwendung der standardisierten Genexpressionsdaten; und
Ausgeben eines Signifikanzwerts der Gene über die mehreren Proben, wobei der Signifikanzwert auf dem Wert der Verteilungsstatistik und der Nullverteilung basiert.

12. Computerimplementiertes Verfahren zum Identifizieren von Genen mit Ausreißer-Expression über mehrere Proben, das Folgendes umfasst:

Empfangen von Genexpressionsdaten einer Vielzahl von Proben, wobei die Proben Genexpressionswerte umfassen, die Genen entsprechen;
Standardisieren der Genexpressionsdaten unter Verwendung des Medians und der mittleren absoluten Abweichung vom Median jedes Gens;
Bestimmen eines Werts einer Verteilungsstatistik für die standardisierten Genexpressionsbeobachtungen basierend auf einer Wahrscheinlichkeit von Ausreißer-Genexpressionsdaten, wobei das Bestimmen des Werts der Verteilungsstatistik Folgendes umfasst: für jede Probe Berechnen einer Wahrscheinlichkeit, dass ein Gen an oder über einem vorbestimmten Schwellenwert exprimiert wird, unter Verwendung von Bernoulli-Versuchen basierend auf der Gesamtanzahl von Proben und eines perzentilen Grenzwerts für das Gen;
Bestimmen einer Nullverteilung der Verteilungsstatistik unter Verwendung der standardisierten Genexpressionsdaten; und
Ausgeben eines Signifikanzwerts der Gene über die mehreren Proben, wobei der Signifikanzwert auf dem Wert der Verteilungsstatistik und der Nullverteilung basiert.

**Revendications**

1. Système de détection permettant d'identifier des gènes présentant une expression aberrante à travers de multiples échantillons, comprenant :

au moins un processeur ; et

au moins un support non transitoire lisible par ordinateur contenant des instructions qui, lorsqu'elles sont exécutées par ledit au moins un processeur, amènent ledit au moins un processeur à mettre en œuvre des opérations comprenant les étapes ci-dessous consistant à :

recevoir des données d'expression génique d'une pluralité d'échantillons, les échantillons comprenant des valeurs d'expression génique correspondant à des gènes ;

normaliser les données d'expression génique en utilisant l'écart médian et l'écart absolu médian de chaque gène ;

déterminer une valeur d'une statistique de distribution pour les observations d'expression génique normalisées sur la base d'une probabilité de données d'expression génique aberrantes, dans lequel l'étape de détermination de la valeur de la statistique de distribution comprend les étapes ci-après consistant à : pour chaque échantillon, calculer une probabilité qu'un gène soit exprimé à ou au-dessus d'un seuil prédéterminé en utilisant des essais de Bernoulli, sur la base du nombre total d'échantillons et d'un seuil en percentile pour le gène ;

déterminer une distribution nulle de la statistique de distribution en utilisant les données d'expression génique normalisées ; et

fournir en sortie une valeur de signification des gènes à travers les multiples échantillons, la valeur de signification étant basée sur la valeur de la statistique de distribution et la distribution nulle.

2. Système de détection selon la revendication 1, dans lequel la statistique de distribution comprend un quantile.

3. Système de détection selon la revendication 1, dans lequel les opérations incluent en outre l'étape consistant à recevoir des valeurs de signification supplémentaires pour le gène, et l'étape consistant à fournir en sortie une valeur de signification révisée pour le gène sur la base des valeurs de signification supplémentaires reçues pour le gène et de la valeur de signification pour le gène.

4. Système de détection selon la revendication 1, dans lequel le système est mis en œuvre selon une architecture distribuée comprenant un contrôleur qui affecte des tâches à des travailleurs.

5. Système de détection selon la revendication 1, dans lequel l'étape de calcul de la probabilité de l'échantillon inclut l'étape consistant à binariser les valeurs selon la formule donnée ci-dessous :

$$P\left[X_{[(N-1)r+1]} \geq k\right] \cong \sum_{s=(N-1)r+1}^{N} \binom{N}{s} p^s (1-p)^{N-s}$$

$$= pbinom(X \geq (N-1)r + 1, p, N)$$

$$= pbeta(P \leq p, (N-1)r + 1, N - (N-1)r)$$

où : $k$ = score COPA de valeurs aberrantes, $N$ = nombre d'échantillons, $r$ = quantile de valeurs aberrantes, et $p$ = probabilité d'un prélèvement > k.

6. Système de détection selon la revendication 1, dans lequel la vraisemblance de signification nulle dépend en outre du fait que le quantile est un quantile supérieur ou un quantile inférieur.

7. Système de détection selon la revendication 1, dans lequel la distribution comprend une distribution binomiale cumulative.

8. Système de détection selon la revendication 1, dans lequel les opérations incluent en outre l'étape consistant à recevoir des valeurs de signification supplémentaires pour le facteur, et l'étape consistant à fournir en sortie une valeur de signification révisée pour le facteur, sur la base des valeurs de signification supplémentaires reçues pour le facteur et de la valeur de signification pour le facteur.

9. Système de détection selon la revendication 1, dans lequel le système est mis en œuvre en utilisant une architecture informatique parallèle.

10. Système de détection selon la revendication 1, dans lequel la distribution comprend une distribution bêta incomplète.

11. Support non transitoire lisible par ordinateur pour identifier des gènes présentant une expression aberrante à travers de multiples échantillons, comprenant des instructions qui, lorsqu'elles sont exécutées par ledit au moins un processeur, amènent ledit au moins un processeur à mettre en œuvre des opérations comprenant les étapes ci-dessous consistant à :

recevoir des données d'expression génique d'une pluralité d'échantillons, les échantillons comprenant des valeurs d'expression génique correspondant à des gènes ;
normaliser les données d'expression génique en utilisant l'écart médian et l'écart absolu médian de chaque gène ;
déterminer une valeur d'une statistique de distribution pour les observations d'expression génique normalisées sur la base d'une probabilité de données d'expression génique aberrantes, dans lequel l'étape de détermination de la valeur de la statistique de distribution comprend les étapes ci-après consistant à : pour chaque échantillon, calculer une probabilité qu'un gène soit exprimé à ou au-dessus d'un seuil prédéterminé en utilisant des essais de Bernoulli, sur la base du nombre total d'échantillons et d'un seuil en percentile pour le gène ;
déterminer une distribution nulle de la statistique de distribution en utilisant les données d'expression génique normalisées ; et
fournir en sortie une valeur de signification des gènes à travers les multiples échantillons, la valeur de signification étant basée sur la valeur de la statistique de distribution et la distribution nulle.

12. Procédé, mis en œuvre par ordinateur, d'identification de gènes présentant une expression aberrante à travers de multiples échantillons, le procédé comprenant les étapes ci-dessous consistant à :

recevoir des données d'expression génique d'une pluralité d'échantillons, les échantillons comprenant des valeurs d'expression génique correspondant à des gènes ;
normaliser les données d'expression génique en utilisant l'écart médian et l'écart absolu médian de chaque gène ;
déterminer une valeur d'une statistique de distribution pour les observations d'expression génique normalisées sur la base d'une probabilité de données d'expression génique aberrantes, dans lequel l'étape de détermination de la valeur de la statistique de distribution comprend les étapes ci-après consistant à : pour chaque échantillon, calculer une probabilité qu'un gène soit exprimé à ou au-dessus d'un seuil prédéterminé en utilisant des essais de Bernoulli, sur la base du nombre total d'échantillons et d'un seuil en percentile pour le gène ;
déterminer une distribution nulle de la statistique de distribution en utilisant les données d'expression génique normalisées ; et
fournir en sortie une valeur de signification des gènes à travers les multiples échantillons, la valeur de signification étant basée sur la valeur de la statistique de distribution et la distribution nulle.

Analysis
System
110

100

Network
130

Data
System
105

User 120A

User Device
120

Fig. 1

Standardize Data    201

Determine Distribution Statistic
Value    203

COPA

202

Determine Null Distribution
(Brute Force Method) 205A

Determine Significance Value
207

Perform Meta-analysis
209

Fig. 2A

```
┌─────────────────────────────────────────────────────────┐
│  ┌─────────────────────────────────┐                     │
│  │   Standardize Data    201       │          COPA       │
│  └─────────────────────────────────┘                     │
│                   │                            202        │
│                   ▼                                       │
│  ┌─────────────────────────────────┐                     │
│  │  Determine Distribution Statistic│                    │
│  │        Value         203        │                     │
│  └─────────────────────────────────┘                     │
└─────────────────────────────────────────────────────────┘
                    │
                    ▼
   ┌─────────────────────────────────┐
   │   Determine Null Distribution   │
   │   (Function Method)  205B        │
   └─────────────────────────────────┘
                    │
                    ▼
   ┌─────────────────────────────────┐
   │  Determine Significance Value   │
   │              207                │
   └─────────────────────────────────┘
                    │
                    ▼
   ┌─────────────────────────────────┐
   │      Perform Meta-analysis      │
   │              209                │
   └─────────────────────────────────┘
```

# Fig. 2B

Standardize Data    201

COPA

202

Determine Distribution Statistic
Value    203

Perform Modified Rank-Product
Test Meta Analysis    600

Fig. 2C

Fig. 3A

Fig. 3B

Fig. 3C

Standardize Data 201

COPA

202

Determine Distribution Statistic Value 203

Determine Null Significance Likelihood 403

Determine Null Significance Threshold 405

Determine Significance Value (Bernoulli Trials) 407

Perform Meta-Analysis 209

Fig. 4A

Fig. 4B

Calculation Method 505

Function Method 503

-log10 (Calculation Method)

-log10 (Function Method)

Fig. 5

600

Receive Analyses    601

Determine Ranks    602

Normalize Ranks    603

Calculate Rank-Products    605

Determine Significance Value    607

Fig. 6

705 Processor

710 Memory

715 Display

Bus

720 I/O Interface

725 Network Adaptor

730

Fig. 7A

700

Caller 735

Controller 740

Worker 745

Fig. 7B

650

A

Normalized Expression (Outlier Gene)

Samples

Normalized Expression (All Genes)

Randomize

versus

8.56

6.30

4.22

6.25

Fig. 8

Fig. 9

EP 3 535 678 B1

Fig. 10

TCGA BRCA

Bootstrap Resampling -log10(*p*)

Random Shuffling -log10(*p*)

Fig. 11

GSE41998 (279) COPA score distribution

TCGA BRCA (1044) COPA score distribution

Fig. 12

EP 3 535 678 B1

TCGA BRCA COPA(95%)

Fig. 14

TCGA BRCA COPA(95%) tail approximation

Fig. 13

Fig. 15

Fig. 16

Fig. 17

Fig. 18

| Graph | Gene Name | Meta Outlier Score | FDR | Product Rank P-value | Median COPA |
|---|---|---|---|---|---|
| 🔍 | ERBB2 | ▱▨▨▨▨ | 0.02 | 2.1E-4 | 3.77 |

Fig. 19A

ERBB2 RNA Expression Study: TCGA - Invasive breast cancer (1043)

☒ Unchanged < **24.64** ≤     ☒ Upregulated     Reference: **normal**

Fig. 19B

Fig. 19C

| Graph | Gene Name | Meta Outlier Score | FDR | Product Rank P-value | Median COPA |
|-------|-----------|--------------------|-----|----------------------|-------------|
| | FAM5C | | 8.7E-7 | 4.9E-11 | 35.66 |
| | PCSK1 | | 8.7E-7 | 4.9E-11 | 5.49 |
| | CD24 | | 1.3E-6 | 2.3E-10 | -3.15 |
| | SCG3 | | 3.5E-6 | 8.0E-10 | 21.44 |
| | CDH1 | | 3.5E-6 | 8.1E-10 | -3.65 |

Fig. 19D

Meta Analysis Heatmap

5 Studies total

Fig. 19E

Fig. 19F

EP 3 535 678 B1

☐ **Cancer**

| | | | | | | | | |
|---|---|---|---|---|---|---|---|---|
| ☐ Malignant tumor of muscle | 83 | **ME** | *RE* | | | | 3 | ⬆up-regulated |
| ☐ Lung cancer | 76 | *RE* | **SM** | **CN** | *MI* | | 15 | ⬇down-regulated |
| ☐ Malignant tumor of intestine | 69 | **ME** | *RE* | **SM** | **CN** | | 33 | ⬇down-regulated |
| ☐ Neuroendocrine tumor | 65 | *RE* | **CN** | | | | 6 | ⬇down-regulated |
| ☐ Brain cancer | 61 | *RE* | **SM** | **CN** | | | 19 | ⬇down-regulated |
| ☐ Breast cancer | 60 | **ME** | *RE* | **CN** | *MI* | | 31 | ⬆up-regulated |
| ☐ Kidney cancer | 53 | *RE* | | | | | 7 | ⬇down-regulated |
| ☐ Multiple myeloma/plasmacytoma | 50 | *RE* | **CN** | | | | 2 | ⬆up-regulated |
| ☐ Prostate cancer | 46 | **ME** *MI* | *RE* | **SM** | **CN** | | 11 | ⬇down-regulated |
| ☐ Liver cancer | 45 | **ME** | *RE* | **CN** | *MI* | | 10 | ⬆up-regulated |

Fig. 19G

$N = 21$
$r = 0.25$

$(N - 1)r + 1$         $N - ((N - 1)r + 1)$

$X_{[1]}$         $X_{[(N - 1)r + 1]}$         $X_{[N]}$

$$P\big[X_{[(N-1)r+1]} \geq k\big] \cong \sum_{i=(N-1)r+1}^{N} \binom{N}{i} p^i (1-p)^{N-i}$$

$$= pbinom(X \geq (N - 1)r + 1, p, N)$$
$$= pbeta(P \leq p, (N - 1)r + 1, N - (N - 1)r)$$

Fig. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SCOTT A. TOMLINS ; DANIEL R. ; RHODES ; SVEN PERNER ; SARAVANA M. DHANASEKARAN ; ROHIT MEHRA ; XIAO-WEI SUN ; SOORYANARAYANA VARAMBALLY ; XUHONG CAO ; JOELLE TCHINDA.** *Recurrent fusion of TMPRSS2 and ETS transcription factor genes in prostate cancer*
- **JEFFREY DEAN ; SANJAY GHEMAWAT.** *MapReduce: Simplified Data Processing on Large Clusters*
- **MATEI ZAHARIA ; MOSHARAF CHOWDHURY ; MICHAEL J. FRANKLIN ; SCOTT SHENKER ; ION STOICA.** *Spark: Cluster Computing with Working Sets*
- **DANIEL R. RHODES ; JIANJUN YU ; K. SHANKER ; NANDAN DESHPANDE ; RADHIKA VARAMBALLY ; DEBASHIS GHOSH ; TERRENCE BARRETTE ; AKHILESH PANDEY ; ARUL M CHINNAIYAN.** *ONCOMINE: A Cancer Microarray Database and Integrated Data-Mining Platform*
- **TOMLINS SA ; RHODES DR ; PERNER S ; DHANASEKARAN SM ; MEHRA R et al.** Recurrent fusion of TMPRSS2 and ETS transcription factor genes in prostate cancer. *Science,* 2005, vol. 310, 644-648
- **RHODES DR ; YU J ; SHANKER K ; DESHPANDE N ; VARAMBALLY R et al.** ONCOMINE: A Cancer Microarray Database and Integrated Data-Mining Platform. *Neoplasia,* 2004, vol. 6 (1), 1-6
- **TIBSHIRANI R ; HASTIE T.** Outlier sums for differential gene expression analysis. *Biostatistics,* 2006, vol. 8, 2-8
- **WU B.** Cancer outlier differential gene expression detection. *Biostatistics,* 2007, vol. 8 (3), 566-575
- **LIAN H.** MOST: detecting cancer differential gene expression. *Biostatistics,* 2008, vol. 9 (3), 411-418
- **DE RONDE JJ ; RIGAILL G ; ROTTENBERG S ; RODENHUIS S ; WESSELS LF.** Identifying subgroup markers in heterogeneous populations. *Nucleic Acids Research,* 2013, vol. 41 (21), e200
- **WANG C ; TACIROGLU A ; MAETSCHKE SR ; NELSON CC ; RAGAN MA et al.** mCOPA: analysis of heterogeneous features in cancer expression data. *J Clin Bioinforma,* 2012, vol. 2, 22
- **RHODES DR ; ATEEQ B ; CAO Q ; TOMLINS SA ; MEHRA R et al.** AGTR1 overexpression defines a subset of breast cancer and confers sensitivity to losartan, an AGTR1 antagonist. *Proc. Natl. Acad. Sci.,* 2009, vol. 106 (25), 10284-10289
- **CHANG LC ; LIN, HM ; SIBILLE E ; TSENG GC.** Meta-analysis methods for combining multiple expression profiles: comparisons, statistical characterization and an application guideline. *BMC Bioinformatics,* 2013, vol. 14, 368
- **EDGINGTON E.** Randomization Tests. Marcel Dekker, Inc, 1980
- **KNIJNENBURG TA ; WESSELS LFA ; REINDERS MJT ; SHMULEVICH I.** Fewer permutations, more accurate P-values. *Bioinformatics,* 2009, vol. 25, 161-168
- **GUMBEL EJ.** Statistics of extremes. Columbia University Press, 1958
- **KUPERSHMIDT I ; SU QJ ; GREWAL A ; SUNDARESH S ; HALPERIN I et al.** Ontology-Based Meta-Analysis of Global Collections of High-Throughput Public Data. *PLoS ONE,* 2010, vol. 5 (9), e13066
- **HESKES T ; EISINGA R ; BREITLING R.** A fast algorithm for determining bounds and accurate approximate p-values of the rank product statistic for replicate experiments. *BMC Bioinformatics,* 2014, vol. 15 (367
- **HYNDMAN RJ ; FAN Y.** Sample quantiles in statistical packages. *The American Statistician,* 1996, vol. 50 (4), 361-365
- **PRESS WH ; FLANNERY BP ; TEUKOLSKY SA ; VETTERLING WT.** Numerical Recipes in C: The Art of Scientific Computing. Press Syndicate of the University of Cambridge, 1992